(19) Europäisches Patentamt
European Patent Office
Office européen des brevets

(11) **EP 2 744 866 B1**

(12) **EUROPÄISCHE PATENTSCHRIFT**

(45) Veröffentlichungstag und Bekanntmachung des Hinweises auf die Patenterteilung:
**03.04.2019 Patentblatt 2019/14**

(51) Int Cl.:
*C09J 7/22* (2018.01)     *A61L 15/44* (2006.01)
*A61L 15/46* (2006.01)     *A61L 15/58* (2006.01)

(21) Anmeldenummer: **12762540.8**

(22) Anmeldetag: **10.08.2012**

(86) Internationale Anmeldenummer:
**PCT/EP2012/065699**

(87) Internationale Veröffentlichungsnummer:
**WO 2013/024032 (21.02.2013 Gazette 2013/08)**

(54) **SELBSTKLEBENDE POLYMERISATIONSPRODUKTE**

SELF ADHESIVE POLYMERISATION PRODUCTS

PRODUITS DE POLYMÉRISATION AUTO-ADHÉSIFS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.08.2011 DE 102011080958**

(43) Veröffentlichungstag der Anmeldung:
**25.06.2014 Patentblatt 2014/26**

(73) Patentinhaber: **Beiersdorf AG**
**20253 Hamburg (DE)**

(72) Erfinder:
• **WÖLLER, Karl-Heinz**
**20257 Hamburg (DE)**
• **HAHN, Janina**
**22926 Ahrensburg (DE)**
• **NIERLE, Jens**
**21073 Hamburg (DE)**

(74) Vertreter: **Hartmann, Jost**
**Beiersdorf AG**
**Unnastrasse 48**
**20253 Hamburg (DE)**

(56) Entgegenhaltungen:
**EP-A2- 0 259 094    DE-C1- 4 303 183**

EP 2 744 866 B1

**Beschreibung**

Die Erfindung ist ein selbstklebendes Polymerisationsprodukt umfassend eine Klebmasse und einen darauf aufgebrachten nicht-klebenden Träger. Der Träger enthält polymerisationsfähige Monomere, Oligomere und/oder Polymere, die teilweise oder vollständig in die Klebmasse einpolymerisiert sind.

Die Polymerisation von Haftklebmassen oder Polymersystemen kann über anionische Polymerisation, kationische Polymerisation und/oder radikalische Polymerisation erfolgen.

Anionische Polymerisation wird bevorzugt bei Monomeren mit elektronenziehenden Substituenten wie Nitril-, Carboxyl-, Phenyl- und Vinylgruppen angewandt. Initiiert wird die anionische Polymerisation durch Basen bzw. LEWIS-Basen, wie z. B. Metallamide (NaNH2), Alkoxide, Hydroxide, Cyanide, Phosphine, Amine, organometallische Verbindungen wie Buthyl-Lithium (n-C4H9Li) oder Grignard-Verbindungen (z. B. Phenylmagnesiumbromid).

Da im idealen Fall anionische Polymerisationen keine Abbruchreaktionen aufweisen, wird die Polymerisation durch Zusätze gestoppt. Solche Zusätze ermöglichen die Einführung interessanter funktioneller Endgruppen wie -COOH, -OH, -NH2 etc.

Technische Bedeutung hat die anionische Polymerisation für die Herstellung von Polyamid 6 (Perlon), Poly(oxymethylen) (POM) und für die Synthese von Poly(Styrol-*block*-Butadien-*block*-Styrol) erlangt.

Kationische Polymerisation findet bei Monomeren mit elektronenschiebenden (+M-Effekt) Substituenten wie Alkoxy-, Phenyl-, Vinyl- und 1,1-Dialkylgruppen statt. Sie läuft meist über Carbenium- und Oxonium-Ionen ab. Aufgrund der hohen Reaktivität und der damit verbundenen Instabilität der Ionen finden viele Nebenreaktionen (Abbruch-, Übertragungsreaktionen) statt, was einen technischen Einsatz enorm einschränkt. Perchlor-, Schwefel-, Phosphor- und Trifluormethansulfonsäure werden dagegen für die kationische Polymerisation verwendet. Das Molekulargewicht der entstehenden Polymere ist allerdings selten höher als 2.000-3.000 g/mol. Eine große Vielzahl von LEWIS-Säuren initiiert die kationische Polymerisation bei niedrigen Temperaturen. Eingesetzt werden können z. B. Metallhalogenide (AlCl3, BF3, SnCl4), Organometallverbindungen (RxAlCl3-x) oder Oxyhalogenide (POCl3, SOCl2,). Der Start der Polymerisation mit LEWIS-Säuren erfordert aber entweder zur Beschleunigung der Reaktion oder generell zur Initiierung Spuren eines Protonendonors (Wasser, Alkohol, organische Säure) oder eines Kationendonors (*tert*-Butylchlorid, Triphenylmethylfluorid). Typische Abbruchreagenzien sind Wasser, Alkohole, Anhydride und auch Ester bzw. allgemein Nucleophile. Technische Bedeutung hat die kationische Polymerisation für die Herstellung von Poly(isobuten), Butylkautschuk, Poly(vinylether)n und Poly(lactid)en erlangt.

Da Radikalreaktionen unspezifisch sind, können mit Hilfe der radikalischen Polymerisation nahezu alle Vinylmonomere polymerisiert werden.

Kleben zählt nach DIN 8593 zu den Fügeverfahren und beschreibt das flächige Verbinden von gleichen oder verschiedenen Stoffen unter Verwendung einer nichtmetallischen Verbindung, welche durch physikalische oder chemische Reaktionen verfestigt bzw. ausgehärtet wird.

Haftklebstoffe verfügen bei Raumtemperatur über eine permanente Klebrigkeit und können durch leichtes Andrücken an eine Oberfläche die zur Verklebung notwendigen Haftkräfte ausbilden.

Man unterscheidet Klebstoffe, die sofort beim aneinander anhaften der Fügeteile zusammenkleben. Im Gegensatz dazu gibt es Reaktionsklebstoffe, bei denen die Fügeteile erst nach einer Reaktionszeit fest aneinander kleben oder Schmelzhaftklebstoffe, die in der Schmelze appliziert werden und erst nach Erkalten die Klebeteile fest verfügen.

Die Haftklebung stellt einen sehr komplexen Vorgang dar und beruht auf dem Zusammenwirken von Adhäsionskräften zwischen den Molekülen der Grenzschichten zweier verschiedener Stoffe und Kohäsionskräften zwischen gleichartigen Molekülen innerhalb des Klebstoffs.

Um eine erfolgreiche Haftung zu erzielen sollten drei Bedingungen erfüllt sein:

- Der Klebstoff muss die Oberfläche des Verbindungspartners benetzen.
- Diese Oberfläche muss adhäsionsfreundliche Wechselwirkungen eingehen können.
- Der Klebstoff muss eine ausreichende innere Festigkeit besitzen.

Um diesen Anforderungen gerecht zu werden bestehen Haftklebstoffe aus hochviskosen Polymersystemen, die im Endzustand teilweise die Eigenschaften einer Flüssigkeit beibehalten und sich Oberflächenkonturen vollständig anpassen können. Zusätzlich besitzen sie durch ihre Festigkeit teilweise die Eigenschaften elastischer Körper und somit die Fähigkeit Kräfte zwischen den Fügeteilen zu übertragen. Diese Kombination wird als viskoelastisches Verhalten bezeichnet.

Wichtige Einsatzgebiete für Haftklebstoffe sind die Verklebung zum Verbund von flächigen Komponenten miteinander, sowie die Verklebung zur Reparatur von Defekten flächiger Komponenten. Hierfür wird der Haftklebstoff während der Herstellung auf einer Seite mit einem nichtklebenden sogenannten Trägermaterial, kurz Träger, verbunden bzw. laminiert. Dieser nichtklebende Träger kann aus Papier, Gewebe, Vlies, Polymerfolie etc. bestehen. Die zu verbindenden bzw. reparierenden Teile werden gegeneinander oder überlappend platziert und dann an der Nahtstelle beidseitig mit der klebenden Seite des Haftklebstoffs auf dem Träger verbunden.

Diese Art der Anwendung von Haftklebstoffen erfolgt technisch mittels Klebebändern, Klebestreifen, Tapes etc. Der Haftklebstoff auf Träger kann dabei endlos auf sich selbst bzw. auf einer Spule aufgerollt, als auch vorgeformt in Stücken jedweder Größe und Flächigkeit vorliegen. Eines der bekanntesten Produkte dieser Ausführungsformen stellt *tesa-film®* dar. Bei einer Darreichungsform in vorgeformten Stücken wird die klebende Seite solch eines Laminats bis zur Anwendung durch einen anti-adhäsiven sogenannten Release-Liner abgedeckt.

Das Analogon zur Verklebung flächiger Defekte in der Wundversorgung stellen selbstklebende Pflaster dar. Wundpflaster bestehen aus einer selbstklebenden Polymermatrix und einem Trägermaterial. Vor der Applikation des Pflasters wird seine klebende Oberfläche mit einem Release-Liner geschützt. Der Release-Liner wird entfernt und die selbstklebende Polymermatrix wird direkt auf die Haut bzw. die Wunde appliziert.

Die im Jahre 1962 von George D. Winter (G. D. Winter, Nature 1962, 193, 293-294.) gewonnene Erkenntnis, dass ein feuchtes Wundmilieu zu einer beschleunigten Wundheilung und zur Reduktion des Infektionsrisikos beiträgt, änderte die Anforderungen an Pflaster. Diese sollen

- ein feuchtes Wundmilieu schaffen und aufrecht erhalten;
- das überschüssige Wundsekret aufnehmen und zurückhalten;
- die Wunde vor äußeren mechanischen Einflüssen und Verunreinigungen schützen;
- atraumatisch und rückstandsfrei entfernbar sein;
- Gasaustausch mit der Umgebung ermöglichen.

Damit der Haftklebstoff eines Pflasters in der Lage ist überschüssiges Exsudat oder Hautfeuchtigkeit aufzunehmen, gibt es zwei verschiedene Prinzipien.

Zum einen können absorbierende Partikel in Form von hydrophilen Kolloiden oder Superabsorbern direkt in die Polymermatrix eingearbeitet werden. Diese bilden unter Flüssigkeitsaufnahme ein hochviskoses Gel und polstern die Wunde aus. Auf diese Weise wird das feuchte Wundmilieu erzeugt und Verklebungen zwischen Pflaster und Wunde vermieden. Ebenso kann eine selbstklebende Polymermatrix auch mit einer absorbierenden Wundauflage versehen werden. Diese ist selbst nicht-adhäsiv, vermeidet so Verklebungen mit der Wunde und schließt das Exsudat sicher ein. Zusätzlich muss die Polymermatrix das für die Anwendung gewünschte und bei wechselnden Umgebungsbedingungen stabile Adhäsions- und Kohäsionsverhalten besitzen.

Den nicht-adhäsiven Abschluss des Pflasters bildet der Träger. Damit ist das Trägermaterial eine zentrale Größe für die Haptik des Pflasters und die Annahme des Produktes beim Konsumenten.

Die Auswahl des Trägers erfolgt primär nach dem Verwendungszweck eines Pflasters. Seine Aufgabe ist es die Wunde vor äußerlicher Kontamination mit Bakterien, Viren oder Wasser zu schützen. Außerdem bestimmt es in hohem Maße die Gaspermeabilität und deren Selektivität. Der Zusammenhalt zwischen der Polymermatrix und dem Träger wird allein durch Adhäsionskräfte an ihrer Grenzfläche erzeugt. Bei modernen Wundauflagen wird häufig eine semi-okklusive Polyurethanfolie verwendet.

Nachteilig bei der Verwendung von Folien jedweder Art als Träger ist, dass der Verbund mit der Haftklebmasse lediglich über das Adhäsionsvermögen der Haftklebmasse selbst erfolgt. Bedingt durch die allgemein geringe Oberflächenrauigkeit von Folien, deren unpolaren Charakter, elektrostatischer Abstoßung zwischen Haftklebmasse und Trägerfolie sowie anderer Grenzflächenphänomene kann es zu Delaminationen zwischen Haftklebstoff und Träger kommen.

Nachteilig bei bekannten Laminaten ist oft, dass die Laminate bei mechanischen Beanspruchungen delaminieren können, was insbesondere bei medizinischen Produkten der Fall sein kann.

EP 0 446 431 A2 offenbart ein Trägermaterial für medizinische Pflaster, das von einem Laminat gebildet wird, das seinerseits aus einer ersten polymeren Filmschicht, einer zweiten, auf der ersten polymeren Filmschicht erzeugten Filmschicht und einer dritten, in der zweiten Schicht teilweise eingebetteten und auf diese Weise darin verankerten Schicht aus einem makroporösen textilen Material, besteht.

Aus WO 97/42922 A1 ist ein Herstellungsverfahren für ein Laminat bekannt, das einseitig mit einer selbstklebenden Beschichtung versehen ist. Dazu wird ein polymerer Film auf einem thermoplastischen Gewebe oder Vlies durch Hitzeeinwirkung aufgeschmolzen, wobei die Hitze nicht vollflächig einwirkt. Auf die Gewebe- oder Vliesseite wird des Weiteren eine selbstklebende Beschichtung aufgetragen, auf die wiederum eine Wundauflage aufgelegt werden kann.

EP 1 064 149 B1 offenbart ein Laminat, das aus zumindest einer ersten Schicht aus einem elastischen Polymerfilm und einer zweiten Schicht aus einem elastischen Vlies besteht, wobei die erste und zweite Schicht vollflächig verbunden sind, wobei Fasern des Vlies vom Polymerfilm umschlossen werden.

Das Vlies und die Folie weisen dafür ähnliche Rückstellkräfte auf, so dass es bei Dehnungen bis deutlich über 100 % zu keiner Delaminierung des Verbundmaterials kommt.

Derartige voll- oder teilflächige Delaminationen treten insbesondere mit zunehmender Lagerzeit der Verbünde auf, z.B. durch geringfügigen Austritt ölhaltiger Additive aus der Haftklebmatrix oder bei Anwendung auf ungleichmäßig geformten oder bewegten Unterlagen, hierbei bedingt durch unterschiedliche mechanische Stressbeanspruchung der Laminatkomponenten bei Ausrichtungsänderung.

Um diesen Nachteil auszugleichen gibt es diverse technische Angänge, wie z.B. CoronaBehandlung der zu laminierenden Seite der Folie oder die Beschichtung der Folie mit einer weiteren an dieser besser haftenden Klebmatrix, welche auch ein besseres Adhäsionsvermögen zu dem eigentlichen Haftklebstoff besitzt. Alle Methoden bedeuten jedoch zusätzlichen Arbeitsaufwand und Kosten, bieten gleichzeitig aber auch keine absolute Gewähr gegen etwaige Delamination des Verbundes.

Ein weiterer negativer Aspekt von selbstklebenden Laminaten aus Haftklebmassen und Polymerfolien ist der "kalte Fluss" vieler Haftklebmassen. Bedingt durch das viskoelastische Verhalten von Haftklebmassen können diese während der Lagerungs- oder Anwendungszeit langsam unter den äußeren Rändern des abdeckenden nichtklebenden Trägers herausfließen und dort mit anderen Materialien, Bekleidung, Staub, Schmutz etc. verkleben. Das ist insbesondere bei Wundpflastern ebenso aus hygienischen wie ästhetischen Gründen unerwünscht, wie auch allgemein keine bestimmungsgemäße Eigenschaft eines selbstklebenden Polymerlaminats.

Als Ausgleich gegen den kalten Fluss der Haftklebmassen kann man das viskoelastische Verhalten durch weitere Additive, wie sehr langkettige Polymere, zu optimieren versuchen, oder die grundlegende Polymermatrix durch Zusatz von Füllstoffen, wie z. B. Siliziumdioxid oder Cellulose, zu verstrammen. Nachteilig ist hierbei der sehr hohe Versuchsaufwand, insbesondere im Hinblick auf Lagerungstabilität, so wie die Tatsache dass die vorbeschriebenen Einflussnahmen in der Regel mit einer eindeutigen Reduktion des Ahäsionsvermögens der Haftklebmasse verbunden sind.

Wünschenswert ist es daher eine selbstklebende Haut- und/oder Wundauflage zur Verfügung zu stellen, deren Verbund zwischen Klebmasse und Trägerfolie verbessert ist und eine Delaminierung verhindert.

DE 3779707 T2 (EP 259 094 A1) offenbart photopolymerisierbare Haftklebebänder, bestehend aus mehreren aneinanderliegenden Schichten, die nicht delaminierbar sind. In einer der offenbarten Ausführungsform ist eine der Schichten nicht-klebend ausgeführt. Die DE 3779707 T2 beschreibt dabei, dass mehrere Klebmassenschichten ineinander polymerisiert werden können, wobei zumindest eine Klebschicht auf einem Träger aufgebracht ist.

Wünschenswert ist es hauchdünne, elastische Hautauflagen zur Verfügung zu stellen, die insbesondere nahezu unsichtbar ausgestaltet werden können.

Weiterhin ist es wünschenswert ein Laminat, als Wund- oder Hautauflage anwendbar, zur Verfügung zu stellen, das die Nachteile des Standes der Technik, wie beispielsweise den "kalten Fluß" vermeidet.

Überraschenderweise konnten die oben beschriebenen Nachteile von Haut- und/oder Wundauflagen, Haftklebebändern, Haftklebestreifen etc. durch Herstellung eines für diese Verwendung geeigneten Polymerisationslaminates überwunden werden.

Die Erfindung ist ein selbstklebendes Polymerisationsprodukt, insbesondere eine Hautauflage, umfassend eine Klebmasse und einen darauf aufgebrachten nicht-klebenden Träger. Der Träger umfasst vor der Polymerisation polymerisationsfähige Monomere, Oligomere und/oder Polymere, die teilweise oder vollständig in die Klebmasse einpolymerisiert sind.

Das selbstklebende Polymerisationsprodukt weist eine maximale Dicke von bis zu 800 μm, insbesondere bis 350 μm und vorteilhaft im Bereich von 20 bis 250 μm auf.

Zur Herstellung eines erfindungsgemäßen selbstklebenden Polymerisationslaminats wird zum Beispiel ein handelsüblicher Haftklebstoff mit ausreichender Anzahl an UV-vernetzbaren sowie chromophoren Gruppen als selbstklebende Basis verwendet und dieser dann, auf der bei späterer Anwendung nicht zur Substrat- bzw. Hautverklebung vorgesehenen Seite, mit einem nicht selbstklebenden Polymer durch freie radikalische Polymerisation in einer gemeinsamen gradientenartigen Übergangsschicht zwischen Haftklebstoff und nicht selbstklebenden Polymer chemisch dauerhaft gebunden.

Die gradientenartige Übergangsschicht, diejenige Schicht der Klebmasse, in der die Monomere, Oligomere und/oder Polymere der Trägerschicht teilweise und/oder vollständig einpolymerisiert sind, weist eine Tiefe von bis zu 100 μm auf. Bevorzugt ist diese Schichttiefe bis zu 50 μm zu wählen.

Bei einem erfindungsgemäßen selbstklebenden Polymerisationslaminat als Endprodukt fungiert das auf und in den Haftklebstoff einpolymerisierte Polymer quasi als Träger und kann von dem Haftklebstoff nur noch durch chemische Umsetzungen, nicht aber durch rein physikalische Einflüsse wieder getrennt werden. Eine Delaminierung durch mechanische Stressbeanspruchung, Dehnung etc. wird vermieden.

Dies ist ein gegenüber den aus dem Stand der Technik offenbarten Verfahren wesentlicher Unterschied. Erfindungsgemäß wird auf eine ggf. vorpolymerisierte Klebschicht eine nichtklebende, polymerisierbare Trägermasse aufgetragen.

Der Träger ist erfindungsgemäß bei der Anwendung des Polymerisationsproduktes, insbesondere der Hautauflage, nach aussen gerichtet. D.h. Die Klebmasse weist zur beklebenden Oberfläche, insbesondere der Haut, und der Träger zur entgegen gesetzten Seite.

Das Verfahren zur Herstellung des selbstklebenden Polymerisationsproduktes erfolgt durch die Schritte der Applikation eines Trägermaterials, umfassend ein oder mehrerer polymerisationsfähige Monomere, Oligomere und/oder Polymere, sowie ggf. weitere Zusatzstoffe, bevorzugt in Lösung, auf eine Schicht eines ggf. teilweise vorvernetzten Haftklebstoffes, umfassend bevorzugt UV-vernetzbare Acrylathaftklebstoffe, Photoinitiatoren sowie ggf. weitere Zusatzstoffe, und Polymerisation der Haftkleb-Trägerschichten bevorzugt mittels UV-Lichtbestrahlung, bevorzugt mit 10 bis 200 mJ/cm$^2$, bevorzugt 120 bis 150 mJ/cm$^2$.

Die Polymerisation der Haftklebemasse als auch der Träger Monomere/Oligomere/Polymere erfolgt bevorzugt radikalisch UV initiiert.

Die Haftklebemasse kann zuerst polymerisiert und anschließend kann auf die obere Seite der Klebschicht der Träger einpolymerisiert werden oder die Polymerisation der Klebmasse und Trägers erfolgt zeitgleich.

Beide Varianten sind möglich. Bevorzugt ist die Haftklebmasse vorpolymerisiert, so dass sie noch fließfähig ist und noch ausreichend Funktionalitäten übrig hat. Man kann diese Matrix dann ausstreichen und weiter vernetzen, sprich weiter polymerisieren, so dass sie praktisch schon die finalen physikalischen Eigenschaften aufweist und danach den Trägerfilm erfindungsgemäß auf- und einpolymerisieren.

Erfindungsgemäß kann auf die gestrichenen Klebmatrix aber auch gleich die Trägerpolymere auftragen werden und dann beides zusammen, idealerweise per UV-Bestrahlung, durchpolymerisiert/vernetzt werden.

Diese Variabilität ermöglicht die konkreten physikalischen Eigenschaften der Träger- bzw. Klebmassenschicht individuell einzustellen und je nachdem wie die Polymerisation in der Produktion umsetzbar ist. Es lassen somit die Klebkraft, Schichtdicke der Haftklebmatrix, Vorvernetzungsgrad der Haftklebmasse und Intensität der UV-Quelle individuell einstellen.

Zur Herstellung erfindungsgemäßer selbstklebender Polymerisationslaminate wird als selbstklebende Basis z.B. ein handelsüblicher Haftklebstoff mit ausreichender Anzahl an UV-vernetzbaren sowie chromophoren Gruppen verwendet. Diese Basis kann dann zur Erzielung der am finalen Produkt gewünschten Eigenschaften mit entsprechenden Zuschlagsstoffen compoundiert werden, wie z.B. in den Beispielrezepturen 1 - 5 beschrieben.

Das Compoundieren kann dabei je nach den chemisch-technologischen Erfordernissen der Haftklebstoffe lösemittelfrei erfolgen, z.B. in einem Kneter oder einem Extruder, ggf. bei Heißschmelzklebstoffen unter Erwärmung bzw. in der Schmelze.

Das Compoundieren kann aber auch als Lösemittelprozess erfolgen. Dabei wird der Haftklebstoff in einem entsprechenden Lösemittel, wie z.B. Ethylacetat oder Benzin, gelöst und anschließend die gewünschten Zuschlagsstoffe unter Rühren oder Kneten eingearbeitet. Der fertig compoundierte Haftklebstoff wird auf einem Trennträger, einer Trennfolie oder Ähnlichem mittels eines Rakels, einer Breitschlitzdüse, durch Kalandrieren oder einer anderen geeigneten Technik in der gewünschten Schichtdicke aufgetragen.

Bei einer in einem Lösemittelprozess hergestellten Haftklebmasse lässt man dann das Lösemittel unter geeigneten, ggf. heizbaren, Abzugs- und Filteranlagen verdunsten.

Zur Erzielung einer größeren Gradiententiefe der Polymerisationsschicht des Trägermaterials mit dem als Basis dienenden Haftklebstoff kann es von Vorteil sein, wenn bei lösemittelhaltigen Haftklebstoffen bzw. Compounds das Lösemittel vor der Zugabe des Trägerpolymerisats nicht oder nur teilweise verdunstet wird. Durch die niedrige Viskosität des Compoundsystems können die den Träger umfassenden polymerisationsfähigen Monomere, Oligomere und/oder Polymere tiefer in den Haftklebstoff eindringen. Je tiefer die Trägerkomponenten gradientär in die Haftklebmasse eindringen, desto mehr funktionelle Gruppen der Trägerkomponente können sich mit den entsprechenden UV-vernetzbaren sowie chromophoren Gruppen der Haftklebmasse verbinden und desto stärker ist die Polymerisationsbindung zwischen Haftklebmasse und Trägerkomponente.

Bevorzugt weist die Eindringtiefe, die gradientenartige Übergangsschicht, einen Wert von 1 bis maximal 100 μm auf.

Ebenfalls kann es von Vorteil sein, je nach gewünschter Klebkraft des finalen Laminats, wenn der wie vorstehend beschriebene Basis Haftklebstoff in Abhängigkeit von der Schichtdicke vor der Laminierung mit dem Trägerpolymerisat mit 10 bis 200 mJ/cm$^2$ UV-Licht vorvernetzt wird, anschließend die so vorbehandelte Seite des Haftklebstoffs mit Trennträger, Trennfolie, Trennpapier abgedeckt wird und die bisherige Unterseite, vom Trennmedium befreit, zur Weiterverarbeitung als erfindungsgemäßes Laminat genutzt wird. Je höher die Vorvernetzung der Haftklebmasse allerdings ist, desto weniger funktionelle Gruppe aus dieser stehen für die spätere Einpolymerisation der entsprechenden Trägerkomponente zur Verfügung. Dadurch erhöht sich der Polymerisationsgrad der Trägerkomponenten untereinander, somit auch die Kohäsivität der Trägerschicht des finalen Polymerisationslaminats, gleichzeitig verringert sich aber der gegenseitige Polymerisationsverbund der beiden Basismaterialien, Haftklebmasse und Trägerkomponente, eines erfindungsgemäßen Polymerisationslaminats.

Auch hier ist eine gewisse Flexibilität und Anpassungsmöglichkeit im Hinblick auf die Anforderungen des Endproduktes gegeben.

Die den Träger umfassenden polymerisationsfähigen Monomere, Oligomere und/oder Polymere sowie ggf. weitere Zusatzstoffe, wie beispielhaft aber nicht ausschließlich unter den Beispielrezepturen 6 - 67 beschrieben, werden unter Rühren bei Raumtemperatur, ggf. unter leichter Erwärmung, in einem organischen Lösemittel, bevorzugt Ethylacetat, gelöst. Der Gehalt an Lösemittel sollte dabei vorteilhaft maximal 50 Gew.-% betragen um eine ausreichende Filmbildung zu gewährleisten.

Diese Lösung der Trägerkomponenten eines erfindungsgemäßen Polymerisationslaminats wird dann mittels der dem Fachmann bekannten Techniken wie Rakeln, Aufsprühen etc. in der gewünschten Schichtdicke auf den wie vorstehend als Basis beschriebene Haftklebstoff appliziert.

Zum Abdunsten des Lösemittels lässt man die Lösung dann 1 bis 30 min., bevorzugt 5 min., auf die Haftklebmasse einwirken. Dabei dringen die Monomere, Oligomere und/oder Polymere sowie ggf. weitere Zusatzstoffe der Trägerkomponente zusammen mit dem Lösemittel entsprechend in die Tiefe der Haftklebmasse ein.

Zur Ausbildung des finalen Polymerisationslaminats wird das Komposit dann mit 10 bis 200 mJ/cm$^2$, bevorzugt 120 bis 150 mJ/cm$^2$ UV-Licht bestrahlt.

Anschließend können dann gewünschte Geometrien des Endprodukts gestanzt oder geschnitten werden, oder gewünschte Breiten geschnitten und auf Trennpapier oder auf der Trägerseite aufgerollt ausgefertigt werden.

Ein weiterer Vorteil dieses Verfahrens liegt darin begründet, dass sich sehr dünne und damit flexible, sich etwaigen Unebenheiten des Substrats sehr gut anschmiegende selbstklebende Laminate herstellen lassen. Theoretisch kann die minimale Schichtdicke des Trägers bei erfindungsgemäßen selbstklebenden Polymerisationslaminaten lediglich eine monomolekulare Schicht des verwendeten nicht selbstklebenden Polymers betragen.

Da die theoretisch minimale Schichtdicke des verwendeten Laminats ebenfalls nur eine monomolekulare Schicht desselben beträgt, können also mit dem entsprechenden Verfahren extrem dünne selbstklebende Polymerisationslaminate hergestellt werden. Dieser Aspekt birgt für rein technisch-apparative Anwendungen reizvolle Möglichkeiten, solcherlei Produkte sind aber sicherlich nicht praktikabel für menschliche Handhabungen wie z. B. für ein Wundpflaster. Trotzdem haben diese dünnen erfindungsgemäßen selbstklebenden Polymerisationslaminate bei Applikation mittels eines leicht entfernbaren Stützträgers oder mittels einer verpackungsseitigen Applikationshilfe, auch in diesem Anwendungsgebiet große Vorteile.

Entsprechend ausgefertigte Wundpflaster können extrem dünn und damit nahezu unsichtbar ausgestaltet sein, so dass das Pflaster an optisch einsehbaren Stellen appliziert, wie Gesicht oder Dekolleté, einem Dritten als Betrachter nicht auffällt und damit keinerlei mögliche Verunsicherung beim Anwender hervorruft. Weiterhin vorteilhaft kann sich ein entsprechend dünnes Pflaster sehr gut der Hauttopographie anpassen, was den allgemeinen Tragekomfort erhöht.

Bevorzugt haben Wundpflaster die aus einem erfindungsgemäßen selbstklebenden Polymerisationslaminat bestehen eine Schichtdicke des Haftklebstoffs zwischen 5 $\mu$m und 200 $\mu$m, besonders bevorzugt zwischen 10 $\mu$m und 150 $\mu$m.

Je nach Anwendungszweck des erfindungsgemäßen Polymerisationslaminates sind die einzelnen Schichtendicken wie folgt zu wählen:

| Schicht | Schichtdicke | | |
|---|---|---|---|
| | | bevorzugt | vorteilhaft |
| gradientäre Einpolymerisationsschicht - gradientenartige | bis zu 100 $\mu$m | bis 50 $\mu$m | 1 bis 20 $\mu$m |
| Übergangschicht zwischen Träger und Klebmasse, in der sowohl Klebmassenals auch Trägerpolymere enthalten sind | | | |
| Trägerschicht (ohne gradientäre Übergangsschicht) | bis zu 200 $\mu$m | 1 - 100 $\mu$m | 1 -20 $\mu$m |
| Klebmassenschicht (ohne gradientäre Übergangsschicht) | bis zu 500 $\mu$m | 10 - 200 $\mu$m | 20- 100 $\mu$m |
| Polymerisationslaminat | bis zu 800 $\mu$m | bis 350 $\mu$m | 22 bis 240 $\mu$m |

Dabei ist es erfindungsgemäß möglich, dass der gesamte Träger in die Haftklebmasse einpolymerisiert ist und damit die Trägerschicht mit der Einpolymerisationsschicht identisch ist.

Die erfindungsgemäße Einpolymerisationsschicht, die Schicht der Klebmasse in der die Monomere, Oligomere und/oder Polymere des Trägers teilweise und/oder vollständig einpolymerisiert sind, weist eine Dicke (Tiefe) von bevorzugt maximal 100 $\mu$m auf. D.h. vom Träger aus in Richtung Klebemasse ist nach 100 $\mu$m kein Bestandteil des Trägers mehr auszumachen.

Vorteilhaft ist oberhalb der Übergangsschicht eine Trägerschicht vorhanden, in der keine Haftklebmasse enthalten ist.

In der Übergangschicht, der Einpolymerisationsschicht, sind sowohl Trägerkomponenten als auch Haftklebkomponenten vorhanden. Vorteilhaft nimmt der Anteil an Trägerkomponenten, also die Anzahl an Trägerkomponenten pro Gesamtzahl Komponenten, in Richtung zur Haftklebmasse ab. Dies ist als gradientärer Übergang zu verstehen.

Die gradientenmäßige Übergangsschicht der Polymerisation zwischen Haftklebstoff und Träger bildet sich vorteilhaft dabei in einer Dicke bis max. 100 $\mu$m, bevorzugt in einer Dicke bis max. 50 $\mu$m aus, insbesondere in einer Dicke von 1 $\mu$m bis 20 $\mu$m, insbesondere 1 $\mu$m bis 5 $\mu$m.

Als Klebmassen werden bevorzugt solche gewählt, die reaktive Gruppen in den Polymerketten besitzen. Diese reaktiven Gruppen sind in der Lage die Vernetzung mit dem einpolymerisierten Träger zu verstärken. Hierbei sind insbesondere Polymere mit ungesättigten Einheiten wie Doppel- oder Dreifachbindungen zu nennen. Darunter fallen zum Beispiel die *cis*-1,4-Polyisoprene, Styrol-Butadien-Kautschuke, Polyester.

Als problematisch kann sich hierbei eine stark unterschiedliche Polarität der Kautschuke und der aufsprühbaren Acrylatschicht sein, die eine Ausbildung des Gradienten verhindern könnte. In einem solchen Fall muss die Polarität der Klebmasse durch Verschneiden mit geeigneten, polaren Materialien angepasst werden. Hierfür eignen sich zum Beispiel Polyacrylsäuren, oder Polyvinylpyrrolidone.

Besonders vorteilhaft als Haftklebstoff zur Herstellung eines erfindungsgemäßen selbstklebenden Polymerisationslaminats haben sich UV-vernetzbare Acrylathaftklebstoffe erwiesen.

Die Klebmasse umfasst daher bevorzugt UV-vernetzbare Acrylathaftklebstoffe oder besteht vollständig daraus. Entsprechende UV-vernetzbare Haftklebstoffe werden z. B. unter den Bezeichnungen Loctite 3751 von Henkel, Liquid UV-Curable Adhesive LC-4200 von 3M™, UV A4110 UV Cure Acrylic Adhesive von Polymark, UV Adhesive XD 4860 von Huntsman, Delo-Photobond[R] 4302 von DELO, acResin® A 260 UV von der BASF AG und Scotch-Weld™ UV Curing Adhesive UV02 von 3M™ kommerziell angeboten.

Repräsentativ für die Gruppe der UV-vernetzbaren Acrylathaftklebstoffe wurde acResin® A 260 UV von der BASF AG ausgewählt. Dieses wasser- und lösemittelfreie Polyacrylat eignet sich speziell zum Einsatz in medizinischen Klebebändern. Im Gegensatz zu konventionellen UV-Acrylaten, aufgebaut aus einer Mischung von Monomeren, Oligomeren und Photoinitiatoren, bestehen die acResin®-Produkte bereits aus polymeren Grundbausteinen und einem copolymerisierten Photoinitiator. Dies verringert deutlich die toxikologische Bedenklichkeit, welche durch Restmonomere und nicht umgesetzte Photoinitiatormoleküle verursacht werden kann.

Erfindungsgemäß sind UV-vernetzbare Klebmassen bevorzugt. Ein zur Polymerisation bzw. Vernetzung notwendiger Photoinitiator ist dabei als Klebmasse mit umfasst.

Chemisch betrachtet besteht acResin® UV aus einer Acrylatpolymerbasis mit copolymerisierten chromophoren Gruppen. Verwendet werden als Photoinitiatoren Acetophenon- oder Benzophenonderivate, welche durch radikalische Copolymerisation in die Polymergruppe eingebaut werden. Seine Konzentration in der Klebmasse beträgt bevorzugt zwischen 0.0003 - 0.01 mol bezogen auf 100 g Polyacrylat (R. Fink, K.-H. Schumacher, U. Düsterwald, U. Erhardt, H. Meyer, Patentschrift DE 10134261A1*, BASF AG, Ludwigshafen, 2003.).

Durch Bestrahlung mit UV-C-Licht mit einer Wellenlänge zwischen 220 - 280 nm wird der Photoinitiator in den angeregten Triplettzustand überführt. Das Molekül besitzt dadurch einen biradikalischen Charakter und vernetzt mit einer räumlich benachbarten Molekülgruppe des Polymergerüsts. Hierbei erfolgt zunächst eine H-Abstraktion, die mit der Übertragung des radikalischen Charakters auf das Polymergerüst einhergeht. Die Radikale rekombinieren und bilden auf diesem Weg die Vernetzung aus.

Je höher die UV-Dosis im UV-C-Bereich, desto mehr Polymere gehen Vernetzungsreaktionen ein und das Molekulargewicht der Matrix steigt. Hierdurch steigt die Kohäsion der Klebmasse, demgegenüber sinken Adhäsion und Tack. Die Adhäsions- und Kohäsionseigenschaften der Klebmasse können durch geeignete Wahl der UV-Dosis kontrolliert eingestellt werden.

Bevorzugt wird als Klebmasse bzw. als Acrylatkomponente der Klebmasse das BASF ACResin258 ® oder ACResin260 ® eingesetzt, das sich dadurch auszeichnet, dass die Acrylatketten bereits einen einpolymerisierten UV-Vernetzer, analog den Massen mit Benzoinacrylat, enthält. Somit treten keine flüchtigen Bestandteile aus der Masse aus und sie ist damit besonders für Anwendungen auf der Haut geeignet.

Um die Kohäsion der erfindungsgemäßen Klebmasse, insbesondere acResin® 260UV, zu erhöhen und ein Umspulen eines erfindungsgemäßen selbstklebenden Polymerisationslaminats beim späteren Entfernen des Produkts vom Substrat zu vermeiden kann die Matrix noch durch Einarbeitung von Füllstoffen verstrammt werden. Neben den in der Polymerverarbeitung allgemein üblichen Materialien eignen sich dafür insbesondere jedwede hydrokolloide Substanzen wie z.B. Siliziumdioxid und Cellulosetypen. Bevorzugt werden diese Füllstoffe in Größenordnungen von 0,1 % bis 15 Gew. %, besonders bevorzugt 1 Gew.% bis 10 Gew.%, bezogen auf die Gesamtmasse des Haftklebstoff, eingesetzt.

Tabelle 1: Beispielrezeptur der Klebmasse:

|  | Komponente | Gew.-% |
|---|---|---|
| Beispiel 1 | acResin® 260UV | 95.00 |

(fortgesetzt)

|  | Komponente | Gew.-% |
|---|---|---|
|  | Siliziumdioxid | 5,00 |
| Beispiel 2 | acResin® 260UV | 90.00 |
|  | Siliziumdioxid | 10,00 |

Ein weiterer vorteilhafter Haftklebstoff zur Herstellung eines erfindungsgemäßen Polymerisationslaminats stellt eine sogenannte Hybridmatrix dar, als Hauptkomponenten bestehend aus acResin® 260UV und *cis*-1,4-Polyisopren. Diese Matrixkombination vereinigt die Vorteile von Isopren und Acrylaten.

- Isoprenklebmatrices: Hohe Kohäsionsfestigkeit und unpolare Grenzfläche
- Acrylatklebmatrices: Hohe Funktionalität und polare Grenzfläche

Zur Erhöhung der Klebkraft wird in diese Hybridmatrix vorteilhaft zusätzlich noch kurzkettiges Polyisobutylen (PIB) in das Polymergerüst eingearbeitet.
Durch einen Acrylat-Klebmassenanteil von mind. 25 % werden ausreichend viele Acrylatfunktionen an der Oberfläche der Klebmasse für die spätere
Polymerisationsvernetzung mit dem Träger zur Verfügung gestellt. Hochmolekulares cis-1,4-Polyisopren bildet das Polymergerüst, in welches zur Erhöhung der Klebkraft das kurzkettigen Polyisobutylen und zusätzlich acResin® 260 UV eingelagert werden.
Die Formulierung erforderte eine Nachvernetzung mit 140 mJ/cm$^2$ um ihre vollständigen Klebstoffeigenschaften auszubilden. Nach dieser Vernetzung trägt auch acResin® 260UV zur Kohäsion der Matrix bei.

Tabelle 2: Beispielrezepturen Hybridmatrix als Klebmasse:

|  | Komponente | Gew.-% |
|---|---|---|
| Beispiel 3 | Polyisobutylen | 15,00 |
|  | Polyisopren | 46,00 |
|  | acResin® 260UV | 39,00 |
| Beispiel 4 | Polyisobutylen | 18,83 |
|  | Polyisopren | 51,19 |
|  | acResin® 260UV | 29,98 |

Zur Verwendung eines erfindungsgemäßen selbstklebenden Polymerisationslaminats als wirkstoffhaltiges Pflaster oder Transdermales Therapeutisches System, TTS, kann die Haftklebstoffkomponente des Laminats mit entsprechenden kosmetischen, dermatologischen oder pharmazeutischen Wirkstoffen versehen werden. Gfs. werden zusätzlich Lösungsvermittler und/oder Penetrationsbeschleuniger für diese Wirkstoffe benötigt. Eventuelle Verluste im Gehalt von lichtempfindlichen Wirkstoffen können dabei durch Herstellungszuschläge ausgeglichen werden.
Bevorzugte Wirkstoffe sind z. B. Capsaicinoide, Aciclovir, Ibuprofen, Glycerylglycoside, Harnstoff, Paeonol, 4-Butyl-resorcinol, Carnitin, Coffein, Diclophenac, Q10 etc.

Tabelle 3: Beispielrezeptur Wirkstoffmatrix als Klebmasse:

|  | Komponente | Gew.-% |
|---|---|---|
| Beispiel 5 | acResin® 260UV | 94,90 |
|  | Siliziumdioxid | 5,00 |
|  | Capsaicin USP | 0,10 |

Die Klebmasse des Polymerisationsproduktes enthält daher vorteilhaft ein oder mehrere Stoffe ausgewählt aus der Gruppe der Füllstoffe, insbesondere Siliziumdioxid und Cellulose, *cis*-1,4-Polyisopren, Polyisobutylen sowie ein oder mehrere kosmetische, dermatologische oder pharmazeutische Wirkstoffe.

Die Herstellung entsprechender Haftklebmatrices kann sowohl als Lösemittelsystem in einem üblichen Mischer, sowie auch lösemittelfrei als Hot-Melt System in einem Kneter oder Extruder erfolgen.

Zur Ausfertigung eines erfindungsgemäßen selbstklebenden Polymerisationsproduktes wird die flächig vorgeformte Haftklebmatrix auf einer Seite mit geeigneten, polymerisationsfähigen Monomeren, Oligomeren oder Polymeren der sich bildenden Trägerschicht versehen und diese dann zur Ausbildung eines nichtklebenden Trägerlaminats zur gradientären Einpolymerisation auf die Haftklebmatrix verbracht.

Als polymerisationsfähig wird erfindungsgemäß verstanden, dass Monomere, Oligomere und/oder Polymere durch anionische -, kationische - und/oder radikalische Polymerisation in Polymere überführt werden. Das heißt, dass die erfindungsgemäß verwendeten Einsatzstoffe des Trägers über entsprechende reaktive Gruppen vor der Polymerisation verfügen müssen, um eine schnelle Polymerisationsreaktion zu ermöglichen. Besonders bevorzugt ist hierbei die radikalische Polymerisationsmethode, da diese eine hohe Reaktionsgeschwindigkeit zeigt und in vielen Fällen ohne zusätzliche Lösemittel und andere Reaktionsmedien auskommt. Im einfachsten Fall können die flüssigen Komponenten des Trägers vermischt und polymerisiert werden. Erforderlich zum Start der Reaktion ist eine Energiequelle (z.B. UV-Licht) und ggf. ein Radikalstarter (z.B. organische Peroxide). Als geeignete Monomere zeigen sich hier besonders Acrylsäurederivate, da diese sehr leicht radikalische Polymerisationsreaktionen eingehen, die durch UV-Licht induziert werden.

Bevorzugt erfolgt die gradientäre Einpolymerisation der Trägerschicht über eine photochemisch induzierte freie radikalische Polymerisation.

Kurzwellige Strahlung mit einer Wellenlänge von 10 nm bis 380 nm wird als UV-Strahlung bezeichnet. Nach DIN 5103 wird sie in folgende drei Bereiche unterteilt:

- UV-A 315 - 380 nm
- UV-B 280 - 315 nm
- UV-C 200 - 280 nm

Wellenlängen unter 200 nm zählen zum Vakuum-UV. Dieser Teil der Strahlung ist nicht in der Lage sich an Luft auszubreiten, da er von Sauerstoff absorbiert wird.

UV-Strahlung wird nach den für die Anwendung benötigten Wellenlängen und der gewünschten Bestrahlungsstärke mit verschiedenen Strahlungsquellen erzeugt. Die präparativ und im industriellen Umfeld geläufigsten Quellen sind Quecksilberdampflampen. Diese Lichtquellen zeichnen sich im Vergleich zu Lampen mit Edelgasfüllung durch eine hohe Lebensdauer und Effizienz im UV-C-Bereich aus. Hierbei werden nach dem im Betrieb in der Lampe entstehenden Dampfdruck Niederdruck- (0,01-133 Pa), Mitteldruck- (0,01-1 MPa) und Hochdruckstrahler (3-10 MPa) unterschieden. Niederdrucklampen emittieren nahezu monochromatisch bei 253,7 nm.

Mit steigendem Dampfdruck kommt es zur Verbreiterung der spektralen Emissionslinien, wodurch Mittel- und Hochdruckstrahler eine spektrale Verteilung der Lichtleistung aufweisen. Besonders bei industriellen Anwendungen, wie der Aushärtung von Lacken, werden heute Mitteldruckstrahler eingesetzt. Diese besitzen deutlich höhere Leistungsdichten als Niederdruckstrahler und eignen sich somit gut zur effektiven Durchhärtung von Lacken. Die Emissionsmaxima der Strahler liegen bei ca. 250 nm, 310 nm und 366 nm.

Der Großteil der bekannten UV-vernetzbaren Beschichtungen gehört zu den radikalisch härtenden Systemen.

Der Verlauf der erfindungsgemäß bevorzugten freien radikalischen Polymerisation kann in drei Phasen unterteilt werden, wobei die Prozesse parallel ablaufen können. Zunächst müssen Radikale gebildet werden. Bei photochemisch induzierten Reaktionen werden hierzu meist Photoinitiatoren eingesetzt. Beim eigentlichen Kettenstart wird der Radikalcharakter auf im System befindliche Nichtradikale übertragen. Nun schließt sich die Kettenwachstumsreaktion an, bis die Reaktion schließlich terminiert wird.

C.E. Hoyle (C.E. Hoyle, Radiation curing of polymeric materials, American Chemical Society, Nr. 417, Washington, 1990, S. 1-16.) beschreibt beispielhaft UV-vernetzbare Beschichtungen, welche aus den folgenden 4 Komponenten entsprechend Tabelle 4 aufgebaut sind.

Tabelle 4: UV-vernetzbare Beschichtungen

| Komponenten in UV-vernetzbaren Beschichtungen | Gew.-% | Einfluss |
|---|---|---|
| Photoinitiator | 1 - 3 % | Polymerisationsstarter |
| Oligomer | 25 - 90 % | Filmbildung und Basiseigenschaften |
| Monomer | 15 - 60 % | Filmbildung und Viskositätseinstellung |
| Additiv | 1 - 50 % | Pigmente, Tenside, Weichmacher |

Die Verarbeitung der Monomerenkomponenten eines Trägers für ein erfindungsgemäßes selbstklebendes Polymerisationsprodukt erfolgt bevorzugt als Lösung. Bevorzugt sind dabei organische Lösemittel wie z.B. Ethylacetat oder Benzin.

Die Lösung mit den Monomerenkomponenten wird dabei durch Sprühen, Aufrakeln oder andere bekannte geeignete Techniken zur Lösungsbeschichtung in der vorgesehenen Schichtdicke auf den flächig ausgefertigten Haftklebstoff verbracht und das Lösemittel anschließend durch Verdunstungsprozesse eliminiert.

Danach kann die photochemisch induzierte freie radikalische Polymerisation dieser Komponenten als gradientär in die Haftmatrix einpolymerisierter Träger beispielsweise per Quecksilbermitteldrucklampe gestartet werden.

Als Trägermassen werden bevorzugt Pentaerythritoltetraacrylate, Butylacrylate, Polyethylenglycoldiacrylate, Pentaerytritholtriacrylate, Polyurethanacrylate sowie Polyesteracrylate oder Mischungen daraus eingesetzt. Auch Silikonacrylate sind mögliche Trägermasse.

Wird eine, wie bevorzugt, radikalische Polymerisation der Trägerkomponenten gewählt und diese mittels UV-Licht initiiert, sind Photoinitiatoren, wie 2,2-Dimethoxy-2-phenyl-acetophenon, Bestandteil der Trägerkomponenten. Eine Polymerisation mittels UV-Licht ist auch ohne den Einsatz von Photoinitiatoren möglich, führt aber zu längeren Reaktionszeiten und geringeren Vernetzungsgraden, somit empfiehlt sich deren Einsatz.

Als bevorzugte und hochfunktionelle Komponente des Trägers für ein erfindungsgemäßes selbstklebendes Polymerisationsprodukt dient Pentaerythritoltetraacrylat (PTetraA). Es ermöglicht den Aufbau eines dreidimensionalen Netzwerkes, welches eine nicht-adhäsiven Oberfläche ausbildet. Vorteilhaft wird Butylacrylat (BA) zur Verringerung der Viskosität der Zusammensetzung und zur Erhöhung der Reaktionsgeschwindigkeit zugesetzt.

Als Photoinitiator wird 2,2-Dimethoxy-2-phenyl-acetophenon (DMPAP) mit einer für UV-vernetzbare Beschichtungen herkömmlichen Konzentration von 2 Gew.-% eingesetzt. Dieser Photoinitiator zeichnet sich durch seine hohe Reaktivität und thermische Stabilität aus.

Zur Herstellung eines erfindungsgemäßen selbstklebenden Polymerisationsproduktes werden bevorzugt die Substanzen der Trägerausbildung in einem Lösemittel, wie bevorzugt Ethylacetat (EtAc), gelöst.

Dabei zeigen Ergebnisse einer Klebkraftmessung auf Stahl, dass sich bis zu einer Verdünnung von etwa 50 % an Lösemittel, bezogen auf die Gesamtmasse der Trägerlösung, ein Träger mit nicht-adhäsiver Oberfläche ausbildet. Bei einem höheren Lösungsmittelanteil (> 55%) weist die Oberfläche des Trägers eine Klebkraft von 0.29 N/cm bis 0.45 N/cm auf. Eine Klebkraft des Trägers ist jedoch idealerweise zu vermeiden, um bei Anwendung als Haut- oder Wundauflage die Anhaftung von Schmutz, Kleidung etc. zu umgehen. Des Weiteren kann ein klebendes Trägermaterial bei Kontakt mit einem weiteren Gegenstand oder einer Hautpartie an diesem stärker anhaften als auf dem vorgesehenen Untergrund, sich dadurch von diesem lösen, ggf. unter Beschädigung der Oberfläche des Untergrunds, und stellt somit kein voll funktionsfähiges Trägermaterial für ein einseitiges Klebematerial wie z.B. ein Wundpflaster, einen Tapeverband oder ein wirkstoffhaltiges Pflaster dar.

Eine mögliche Erklärung für diesen Sachverhalt ist, dass bei einer Verdünnung von über 50 % die intermolekularen Abstände der auf die Haftklebmasse aufgetragenen Substanzen zu groß sind. Dies hemmt den Diffusionsprozess der reaktiven Komponenten zueinander und limitiert dadurch den Polymerisationsvorgang. Schließlich führt dies zu einem geringen Polymerisationsgrad. Ferner ist die Anzahl der zur Polymerisation verfügbaren Monomere begrenzt, was ebenfalls nur geringe Polymerisationsgrade ermöglicht.

Die Funktionalität der verwendeten Monomere oder Oligomere des Trägers beeinflusst die Dimension und den Polymerisationsgrad der sich bildenden Polymere und Netzwerke und besitzt daher einen Einfluss auf die Eigenschaften des auspolymerisierten Trägers. Folglich ist auch das Verhältnis von mono- zu polyfunktionellem Monomer ein wesentlicher Faktor für die Netzwerkbildung im Träger.

Hierbei gilt je höher der Vernetzungsgrad, desto geringer die Klebkraft des Trägers. Erwartungsgemäß nimmt die Klebkraft des Trägers mit steigendem PTetraA-Gehalt ab, da der Vernetzungsgrad zunimmt. Ab einem Verhältnis der Massenanteile von BA zu PTetraA von 1:1 bildet sich eine nicht-adhäsive Oberfläche. In diesem Fall kommen ca. 2.7 Moleküle BA auf 1 Molekül PTetraA. Bei einem höheren Anteil an monofunktionellen Acrylat kann der zur Ausbildung einer nicht-adhäsiven Oberfläche erforderliche Polymerisationsgrad nicht erreicht werden.

Der Träger des erfindungsgemäßen Polymerisationsproduktes ist nicht klebend formuliert.

Der erfindungsgemäße Träger weist daher eine Klebkraft von weniger als 0,3 N/cm auf um erfindungsgemäß als nicht-klebend zu gelten, idealerweise ist die Klebkraft auf Stahl oder auf Polyethylensubstraten kleiner 0,2 N/cm.

Tabelle 5: Beispielrezepturen für Monomerlösungen der Trägerkomponente (alle Angaben in Gew.-%)

|  | EtAc | BA | PTetraA | DMPAP |
|---|---|---|---|---|
| Beispiel 6 | 50.00 | 39.20 | 9.80 | 1.00 |
| Beispiel 7 | 50.00 | 36.77 | 12.23 | 1.00 |
| Beispiel 8 | 50.00 | 35.00 | 14.00 | 1.00 |

(fortgesetzt)

|  | EtAc | BA | PTetraA | DMPAP |
|---|---|---|---|---|
| Beispiel 9 | 50.00 | 34.33 | 15.17 | 1.00 |
| Beispiel 10 | 50.00 | 32,67 | 16.33 | 1.00 |
| Beispiel 11 | 50.00 | 24.50 | 24.50 | 1.00 |
| Beispiel 12 | 50.00 | 16.33 | 32.67 | 1.00 |
| Beispiel 13 | 50.00 | 25.00 | 25.20 | 0.00 |
| Beispiel 14 | 50.00 | 24.97 | 24.96 | 0.07 |
| Beispiel 15 | 50.00 | 24.87 | 24.86 | 0.27 |
| Beispiel 16 | 50.00 | 24.75 | 7.75 | 0.50 |
| Beispiel 17 | 50.00 | 23.75 | 23.75 | 2.50 |
| Beispiel 18 | 50.00 | 22.50 | 22.50 | 5.00 |

Restmonomere besitzen ein toxikologisches Potential und sollten folglich im Produkt vermieden werden. Einen bedeutenden Beitrag zur unvollständigen Umsetzung der Monomere besitzt die Sauerstoffinhibition. Üblicherweise wird eine freie radikalische Polymerisation unter Raumluftbedingungen durchgeführt. Dies bedingt eine hohe Sauerstoffkonzentration vor allem in der Grenzfläche von Luft und Monomerlösung. Durch die rasche Diffusion von Sauerstoff in tiefere Schichten kommt es auch hier zur Inhibition der Polymerisation. Untersuchungen konnten zeigen, dass Thiole die Sauerstoffinhibition bei der radikalischen Polymerisation verringern. Thiole agieren dabei als Radikalüberträger. Dementsprechend sind sie in der Lage Peroxyradikale wieder in kohlenstoffzentrierte Radikale zu überführen. Die Schwefelverbindung wird während dieses Prozesses in das Polymergerüst eingebaut. Der Übertragungsprozess kann solange ablaufen, bis der Sauerstoff größtenteils aus dem System entfernt ist und die eigentliche Polymerisation ablaufen kann.

Die Arbeit von C. Nason et al. (C. Nason, T. Roper, C. Hoyle, J.A. Pojman, Macromolecules 2005, 38, 5506-5512.) zeigt, dass Trimethylpropantris(3-mercaptopropionat) zur Reduktion der Sauerstoffinhibition bei der UV-induzierten Polymerisation der Trägerkomponente eines erfindungsgemäßen selbstklebenden Polymerisationslaminats eingesetzt werden kann.

Zur Analyse des Einflusses des Thiols auf den Restmonomerengehalt wurden vier Einzelversuche durchgeführt. Hierbei wurden zwei unterschiedliche Thiol-konzentrationen von 1 Gew.-% und 5 Gew.-% verwendet. Die Vernetzung erfolgte mit UV-Dosen von 80 mJ/cm$^2$ und 140 mJ/cm$^2$. Dabei bildeten sich Systeme, die bei einer Bestrahlung mit 140 mJ/cm$^2$ eine nicht-adhäsive Oberfläche und keinen charakteristischen Acrylat- sowie Thiolgeruch aufwiesen. Auch im Fall der Bestrahlung mit 80 mJ/cm$^2$ konnte kein charakteristischer Geruch der Systeme festgestellt werden.

Bei hohen Thiolkonzentrationen ist der Vernetzungsgrad kleiner, da bei der Polymerisation weniger hochmolekulare Bestandteile entstehen. Der charakteristische Acrylatgeruch kann auch in diesen Mustern jedoch nicht mehr nachgewiesen werden. Dies zeigt eindeutig, dass der Monomerenumsatz gesteigert und folglich mehr Monomere zu Oligomeren und Polymeren mit geringen und mittleren Vernetzungsgraden umgesetzt wurden.

Tabelle 6: Beispielrezepturen für Monomerlösungen des Trägers mit der Trägerkomponente Thiol (alle Angaben in Gew.-%)

|  | EtAc | BA | PTetraA | DMPAP | Thiol |
|---|---|---|---|---|---|
| Beispiel 19 | 50 | 23.34 | 23.33 | 1.00 | 2.33 |
| Beispiel 20 | 50 | 24.17 | 24.17 | 1.00 | 0.66 |

Es ist demnach bevorzugt bei radikalischer Polymerisation der Trägermasse den Trägerkomponenten Thiole zu zusetzen.

Mögliche einsetzbare Thiole sind Poly-thiol-Verbindungen, wie beispielsweise Polyoxyalkylentriole mit Mercaptan-Endgruppen, Ester von Mercaptocarbonsäuren mit mindestens trifunktionellen Alkoholen, insbesondere Trimethylpropantris(3-mercaptopropionat), Trimethylolpropan-tris-mercaptoundecanoat , Pentaerythrittetrakismercaptoundecanoat und/oder Tris-mercaptoethylisocyanurat. Untersuchungen zeigten weiterhin, dass durch den Einsatz von acrylatfunktionalisierten Oligomeren die Flexibilität und Elastizität der Trägerbeschichtung verbessert bzw. erst erzeugt werden kann. Zusätzlich reduzieren Oligomere die Volumenschrumpfung während der Polymerisation und optimieren so die Performance des Produktes.

Daher können als entsprechende acrylatfunktionalisierte Oligomere z.B. Polyethylenglycoldiacrylat (PEG-DiA), Pentaerytritholtriacrylat (PTriA), Polyurethanacrylate wie Desmolux U200 und XP2491, sowie Polyesteracrylate wie Desmolux XP 2477(Hersteller BAYER) bevorzugt als Trägerkomponenten eingesetzt werden.

Die Festigkeit der Systeme nimmt ausnahmslos mit steigender Funktionalität der Monomere zu. Besonders ausgeprägt ist der Anstieg allgemein zwischen dem mono-funktionellen BA und dem difunktionellen PEG-DiA. Die Zugfestigkeiten steigen hier durchschnittlich um das 10-fache. Im Fall der beiden verschiedenen Urethanacrylate sind die Verläufe und die erreichbaren Festigkeiten vergleichbar. Bei Verwendung des Polyesteracrylats können viel höhere Festigkeiten der Systeme erreicht werden. Damit einher geht jedoch der Verlust der Elastizität und Flexibilität der erfindungsgemäßen Trägersysteme.

Tabelle 7: Beispielrezepturen für Monomerlösungen der Trägerkomponente acrylatfunktionalisierte Oligomere enthaltend (alle Angaben in Gew.-%)

| | EtAc | BA | PEG-DiA | PTriA | PTetraA | U200 | XP 2491 | XP 2477 | DMPAP |
|---|---|---|---|---|---|---|---|---|---|
| Beispiel 21 | 50.0 | 7.0 | | | 7.0 | 35.0 | | | 1.0 |
| Beispiel 22 | 50.0 | 12.0 | | | 12.0 | 25.0 | | | 1.0 |
| Beispiel 23 | 50.0 | 18.6 | | | 18.7 | 12.7 | | | 1.0 |
| Beispiel 24 | 50.0 | 22.0 | | | 22.0 | 5.0 | | | 1.0 |
| Beispiel 25 | 50.0 | 23.1 | | | 23.2 | 2.7 | | | 1.0 |
| Beispiel 26 | 50.0 | 7.5 | | | 7.5 | | 35.0 | | 1.0 |
| Beispiel 27 | 50.0 | 12.0 | | | 12.0 | | 25.0 | | 1.0 |
| Beispiel 28 | 50.0 | 18.2 | | | 18.1 | | 12.7 | | 1.0 |
| Beispiel 29 | 50.0 | 22.0 | | | 22.0 | | 5.0 | | 1.0 |
| Beispiel 30 | 50.0 | 23.1 | | | 23.2 | | 2.7 | | 1.0 |
| Beispiel 31 | 50.0 | 14.0 | | | 14.0 | | | 35.0 | 1.0 |
| Beispiel 32 | 50.0 | 12.0 | | | 12.0 | | | 25.0 | 1.0 |
| Beispiel 33 | 50.0 | 18.6 | | | 18.7 | | | 12.7 | 1.0 |
| Beispiel 34 | 50.0 | 22.0 | | | 22.0 | | | 5.0 | 1.0 |
| Beispiel 35 | 50.0 | 23.2 | | | 23.1 | | | 2.7 | 1.0 |
| Beispiel 36 | 50.0 | | | 24.5 | | 24.5 | | | 1.0 |
| Beispiel 37 | 50.0 | 24.5 | | | | 24.5 | | | 1.0 |

(fortgesetzt)

| | EtAc | BA | PEG-DiA | PTriA | PTetraA | U200 | XP 2491 | XP 2477 | DMPAP |
|---|---|---|---|---|---|---|---|---|---|
| Beispiel 38 | 50.0 | | 24.5 | | | 24.5 | | | 1.0 |
| Beispiel 39 | 50.0 | | | | 24.5 | 24.5 | | | 1.0 |
| Beispiel 40 | 50.0 | | | 24.5 | | | 24.5 | | 1.0 |
| Beispiel 41 | 50.0 | 24.5 | | | | | 24.5 | | 1.0 |
| Beispiel 42 | 50.0 | | 24.5 | | | | 24.5 | | 1.0 |
| Beispiel 43 | 50.0 | | | | 24.5 | | 24.5 | | 1.0 |
| Beispiel 44 | 50.0 | | | 24.5 | | | | 24.5 | 1.0 |
| Beispiel 45 | 50.0 | 24.5 | | | | | | 24.5 | 1.0 |
| Beispiel 46 | 50.0 | | 24.5 | | | | | 24.5 | 1.0 |
| Beispiel 47 | 50.0 | | | | 24.5 | | | 24.5 | 1.0 |

Um die Elastifizierung der Trägerkomponente eines erfindungsgemäßen selbstklebenden Polymerisationsproduktes bei guter Festigkeit zu erreichen, können Polyethylenglycoldiacrylate (PEG-DiAs) mit unterschiedlichen Molekulargewichten eingesetzt werden. Mit steigendem Molekulargewicht erhöht sich die Kettenlänge der Monomere und damit einhergehend der Abstand zwischen den reaktiven Acrylatgruppen. Hierbei werden reaktive Komponenten verwendet, welche direkt chemisch in das Netzwerk der Trägerkomponente eingebaut werden. Auf diese Weise beeinflussen sie die Eigenschaften der gesamten Formulierung.

Diese Trägerkomponenten-Formulierungen weisen eine gute Stabilität und eine gewisse Elastizität aus. Die Stabilität sowie die Elastizität nehmen mit zunehmender Kettenlänge der PEG-DiAs ab. In diesen Systemen steigt der Abstand der reaktiven Zentren und folglich sinkt die Vernetzungsdichte. Indessen steigt mit der Kettenlänge zwischen den Vernetzungspunkten auch die molekulare Beweglichkeit. Mit zunehmender Kettenlänge steigt also der chemisch für die Polymerisation inerte Charakter der PEG-DiAs.

Tabelle 8: Beispielrezepturen für Monomerlösungen der Trägerkomponente PEG-DiAs unterschiedliche Molekulargewichte enthaltend (alle Angaben in Gew.-%)

| | EtAc | BA | PTetraA | PEG-DiA | | | DMPAP |
|---|---|---|---|---|---|---|---|
| | | | | 258 g/mol | 575 g/mol | 700 g/mol | |
| Beispiel 48 | 50.0 | 22.5 | 22,5 | 3.0 | | | 2.0 |
| Beispiel 49 | 50.0 | 22.5 | 22,5 | | 3.0 | | 2.0 |
| Beispiel 50 | 50.0 | 22.5 | 22,5 | | | 3.0 | 2.0 |
| Beispiel 51 | 50.0 | 9.0 | 9.0 | 30.0 | | | 2.0 |
| Beispiel 52 | 50.0 | 9.0 | 9.0 | | 30.0 | | 2.0 |
| Beispiel 53 | 50.0 | 9.0 | 9.0 | | | 30.0 | 2.0 |

Die Volumenschrumpfung in UV-vernetzbaren Acrylatbeschichtungen bei der Polymerisation kann durch den Zusatz von Füllstoffen deutlich reduziert werden. Die Füllstoffe reduzieren den Monomerenanteil pro Volumeneinheit und bleiben selbst während der Polymerisation unverändert. So können Performanceverluste des Polymersystems aufgrund von Spannungen vermieden werden. Als anorganischer Füllstoff hat sich Siliziumdioxid in Form von pyrogener Kieselsäure in der Beschichtungstechnologie bewährt.

Direkt nach der Polymerisation zeigten sich weniger Spannungen im System, die ansonsten durch den Volumenschrumpf hervorgerufen wurden.

Die Ergebnisse von Zugversuchen zeigen, dass die Festigkeit der Systeme nachweislich ab einem $SiO_2$-Anteil von 0.1 Gew.-% deutlich höher ist als bei der Basisformulierung ohne $SiO_2$. Eine weitere Steigerung der Festigkeit des Systems bei höheren $SiO_2$-Zusätzen ist nicht zu beobachten.

Ein Anteil zwischen 0,1 und 0,25 Gew.% $SiO_2$, bezogen auf die Gesamtmasse des Trägers, ist daher bevorzugt.

Der verstärkende Effekt der Füllstoffpartikel auf die Eigenschaften des Trägerkomponenten-Systems basiert auf der Wechselwirkung Füllstoff-Polymernetzwerk. Diese treten auf, wenn die spezifische Oberfläche der Partikel vollständig von Polymerketten umlagert wird. Die Partikel können im Bereich der Konzentration von 0,1 - 0,25 Gew.-% das bis zu einer Konzentration von 0,1 Gew.-% freie Volumen zwischen den Polymerketten einnehmen und somit deren Festigkeit durch verstrammen erhöhen.

Das freie Volumen ist ab dieser Konzentration gesättigt, wodurch die Festigkeit durch weitere Zugabe von Partikeln nicht gesteigert wird. Die Partikel werden durch das Polymernetzwerk und Füllstoff-Füllstoff-Wechselwirkungen fest im System verankert. Dabei tragen sie zur Mattierung der Oberfläche und folglich Änderung der Haptik bei.

Tabelle 9: Beispielrezepturen für Monomerlösungen der Trägerkomponente unterschiedliche Anteile Siliziumdioxid enthaltend (alle Angaben in Gew.-%)

|  | EtAc | BA | PTetraA | DMPAP | $SiO_2$ |
|---|---|---|---|---|---|
| Beispiel 54 | 48.50 | 24.27 | 24.27 | 1.96 | 1.00 |
| Beispiel 55 | 46.57 | 23.26 | 23.27 | 1.90 | 5.00 |
| Beispiel 56 | 44.15 | 22.02 | 22.02 | 1.80 | 10.01 |
| Beispiel 57 | 36.76 | 18.37 | 18.37 | 1.50 | 25.00 |
| Beispiel 58 | 24.49 | 12.26 | 12.27 | 1.00 | 49.98 |
| Beispiel 59 | 49.00 | 24.49 | 24.50 | 2.00 | 0.01 |
| Beispiel 60 | 48.97 | 24.47 | 24.46 | 2.00 | 0.10 |
| Beispiel 61 | 48.98 | 24.49 | 24.50 | 2.00 | 0.03 |
| Beispiel 62 | 48.77 | 24.37 | 24.36 | 2.00 | 0.50 |

Bei den Trägersystemen mit mehr als 0,25 Gew.% $SiO_2$ lassen sich zwar die Festigkeiten, wie dargestellt, nicht mehr beeinflussen, aber man kann die Oberflächeneigenschaften (Mattigkeit, Farbe) einstellen. Damit sind auch $SiO_2$-Anteile oberhalb 0,25 Gew.%, insbesondere oberhalb 1 Gew.%, bezogen auf die Gesamtmasse des Trägers, möglich.

Eine weitere Möglichkeit Elastizität und Festigkeit der Trägerkomponente erfindungsgemäßer selbstklebender Polymerisationsprodukte zu beeinflussen bildet die Verwendung von Silikonacrylaten. Solche Silikonacrylate können beispielsweise unter der Handelsnamenserie Tego RC von EVONIK gewerblich bezogen werden.

Tabelle 10: Beispielrezepturen für Monomerlösungen der Trägerkomponente unterschiedliche Silikonacrylate enthaltend (alle Angaben in Gew.-%)

|  | EtAc | BA | PEG-DiA | $SiO_2$ | Tego | | | DMPAP |
|---|---|---|---|---|---|---|---|---|
|  |  |  | 258 g/mol |  | RC 706 | RC 709 | RC 902 |  |
| Beispiel 63 | 50.0 | 16.0 | 15.0 | 1.0 | 16.0 |  |  | 2.0 |
| Beispiel 64 | 50.0 | 16.0 | 15.0 | 1.0 |  | 16.0 |  | 2.0 |

(fortgesetzt)

| | EtAc | BA | PEG-DiA | SiO$_2$ | Tego | | | DMPAP |
|---|---|---|---|---|---|---|---|---|
| | | | 258 g/mol | | RC 706 | RC 709 | RC 902 | |
| Beispiel 65 | 50.0 | 16.0 | 15.0 | 1.0 | | | 16.0 | 2.0 |

Tabelle 11: Beispielrezepturen für besonders bevorzugte Monomerlösungen der Trägerkomponente erfindungsgemäßer selbstklebender Polymerisationslaminate (alle Angaben in Gew.-%)

| | EtAc | BA | PEG-DiA | Desmolux | SiO$_2$ | Cellulose | DMPAP |
|---|---|---|---|---|---|---|---|
| | | | 258 g/mol | U 200 | | | |
| Beispiel 66 | 50.0 | 16.0 | 15.0 | 16.0 | 1.0 | | 2.0 |
| Beispiel 67 | 50.0 | 16.0 | 15.0 | 16.0 | | 1.0 | 2.0 |

Zur Charakterisierung des Gesamtsystems erfindungsgemäßer selbstklebender Polymerisationslaminate wurden die Gelwerte einer Probe exemplarisch laminiert aus Beispielrezeptur 1 als Haftklebmasse und Beispielrezeptur 11 als Träger ermittelt.

Der Gelwert gibt den prozentualen Anteil der unlöslichen Bestandteile einer Klebmasse an und lässt somit Rückschlüsse auf den Vernetzungsgrad zu. Die Bestimmung des Gelwertes erfolgt durch Extraktion einer zuvor exakt eingewogenen Probenmenge in einem geeigneten Lösungsmittel über einen definierten Zeitraum. Abhängig vom Vernetzungsgrad und der Art des gewählten Lösungsmittels quellen Polymere hohen Polymerisationsgrades bei Lösungsmittelkontakt an und formen ein Gel. Der Grad der Quellung nimmt mit steigendem Vernetzungsgrad des Polymers ab. Unvernetze und gering vernetzte Polymere gehen mit der Zeit in viskose Lösungen über. Bei vernetzten Polymeren ist die Quellung hingegen begrenzt, d.h. sie sind auch bei höherem Lösungsmittelzusatz unlöslich. Niedermolekulare Komponenten werden vom Lösungsmittel direkt in Lösung überführt.

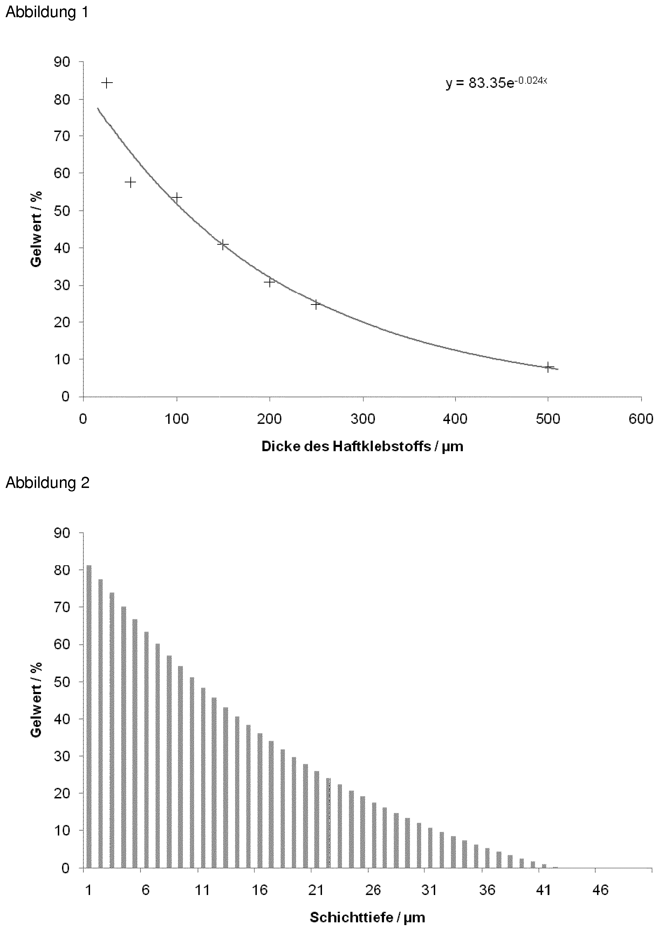

Die Ergebnisse der Messungen werden in der Abbildung 1 zusammengefasst und zeigen dass der Gelwert mit steigender Schichtdicke des Haftklebstoffanteils des Polymerisationslaminats abnimmt.

Unter Verwendung der experimentellen Werte kann für ein erfindungsgemäßes System laminiert aus Beispielrezeptur 1 und Beispielrezeptur 11 hierbei folgender exponentieller Zusammenhang gefunden werden.

$$GW = 83{,}35e^{-0{,}024*DH}$$

GW: Gelwert in % DH: Dicke des Haftklebstoffanteils in $\mu$m

Mit Hilfe dieser Gleichung ist es möglich den Gelwert numerisch für jede einzelne Schicht des Haftklebstoffes zu bestimmen und so Rückschlüsse auf einen möglichen Vernetzungsgradienten im System zu ziehen. Hierzu wird angenommen, dass ein erfindungsgemäßes selbstklebendes Polymerisationslaminatsystem aus n-unabhängigen Schichten mit einer Dicke von je 1 $\mu$m aufgebaut ist. Eine 5 $\mu$m dicke Probe besteht demnach aus 5 einzelnen Schichten. Die Bestimmung des Gelwertes jeder einzelnen Schicht erfolgt im Differenzverfahren nach folgendem Schema:

Oberfläche der Trägerkomponente

$$GW_{S_1} = GW_1 = 83.35e^{-0.024*1}$$

$$GW_{S_2} = 2*GW_2 - GW_{S_1}$$

$$GW_{S_3} = 3*GW_3 - GW_{S_1} - GW_{S_2}$$

$$GW_{S_4} = 4*GW_4 - GW_{S_1} - GW_{S_2} - GW_{S_3}$$

$$GW_{S_5} = 5*GW_5 - GW_{S_1} - GW_{S_2} - GW_{S_3} - GW_{S_4}$$

Der für eine Schicht bestimmte Gelwert wird als Konstante angenommen. Die Abnahme des Gelwertes der Gesamtsysteme erklärt sich durch die stetige "Verdünnung" der hochvernetzten oberen Schichten durch Schichten mit geringem Gelwert.

Die Abbildung 2 zeigt das Ergebnis der numerischen Bestimmung der Gelwerte für die einzelnen Schichten eines erfindungsgemäßen selbstklebenden Polymerisationslaminats bestehend aus Beispielrezeptur 1 (Klebmasse) und Beispielrezeptur 11(Träger). Der Verlauf in Abbildung 2 zeigt den numerisch bestimmten Gelwert der einzelnen Schichten über die Schichttiefe. Hier zeigt sich erwartungsgemäß eine exponentielle Abnahme des Gelwertes der Schichten mit zunehmender Schichttiefe im System. Unlösliche Anteile die zum Gelwert beitragen bilden sich bis in eine Tiefe von 42 $\mu$m. Die Messungen bestätigen dass ein Polymerisationsgradient zwischen der Trägerkomponente und der Haftklebmasse eines erfindungsgemäßen Polymerisationslaminats existiert. Dieser Polymerisationsgradient führt zur erfindungsgemäßen Einpolymerisation der Trägermonomere in die Haftklebmasse und somit zu den erfindungsgemäßen Vorteilen, der aus diesem Polymerisationsprodukt herstellbaren Haut- und/oder Wundauflagen, Haftklebebändern oder Haftklebestreifen.

Abbildung 1 zeigt einen Gelwert 10 bei Dicke der Klebschicht 500 $\mu$m

Die Abbildung zeigt anhand der Messwerte die mit der Schichtdicke abnehmende Vernetzung durch einen exponentiell abnehmenden Gelwert.

Abbildung 2 zeigt einen Gelwert 10 bei Schichtdicke 32 $\mu$m

Der berechnete Gelwert in den unterschiedlichen Schichtdicken zeigt über einen Bereich von 32 $\mu$m das, anhand der experimentell (Abb. 1) ermittelten Daten, zu erwartende Verhalten einer abnehmenden Vernetzung bei zunehmendem Gelwert.

Um die erfindungsgemäße Polymerisationsverankerung des Polymerisationsträgers mit der Haftkleblebmasse sichtbar zu machen wurden rasterelektronenmikroskopische Untersuchungen durchgeführt. Die Untersuchungen erfolgten am Querschnitt der Gefrierbrüche der Proben.

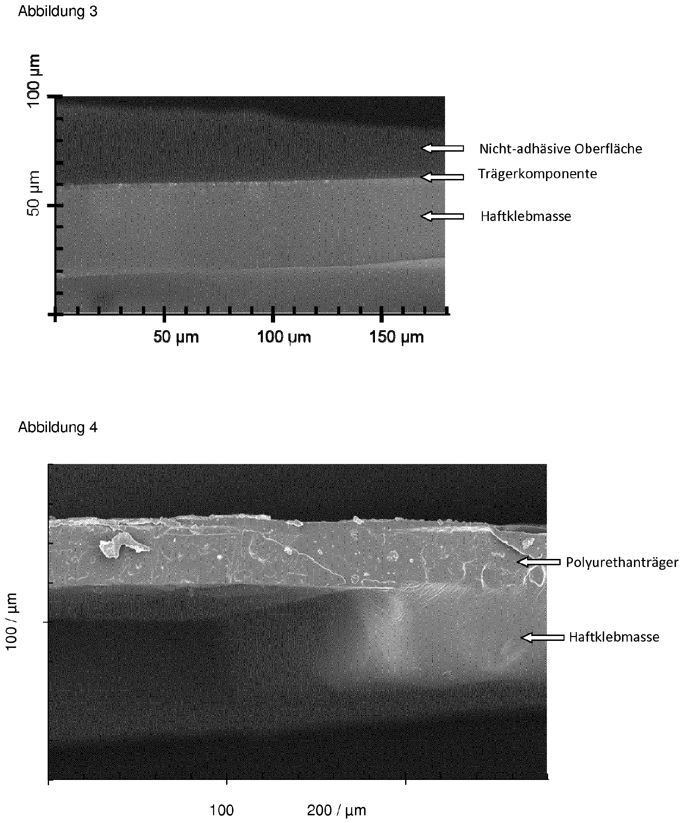

Die Abbildung 3 zeigt die REM-Aufnahme des gesamten Querschnittes eines einpolymerisierten Laminatverbunds aus Beispielrezeptur 1 und Beispielrezeptur 11 nach einer Vernetzung mit 140 mJ/cm$^2$.

Die glatte Oberflächenstruktur der Klebmasse wird vorteilhaft durch Siliziumdioxidpartikel hervorgerufen. Die Oberfläche der reinen Trägerkomponente an der Oberfläche der Klebmasse ist vollkommen glatt.

Um das erfindungsgemäße System mit einem konventionellen Pflaster vergleichen zu können wurde die Klebmasse acResin® 260UV mit einem aufkaschierten Polyurethanträger untersucht. In der Abbildung 4 kann hier schon bei der Betrachtung des gesamten Gefrierbruchs eine deutliche Grenze zwischen den beiden Materialien nachgewiesen werden. Abbildung 4 zeigt die REM-Aufnahme am Gefrierbruch eines Pflastersystems aus acResin® 260UV und einem PU-Träger.

Die Abbildungen der Gesamtsysteme heben die unterschiedlichen Dimensionen der Trägermaterialien hervor. Während die einpolymerisierte Trägerschicht nur wenige $\mu$m dick ist, besitzt der verwendete PU-Träger der Abbildung 4 eine Dicke von ca. 40 $\mu$m.

Die Übergänge zwischen Träger und Klebmasse können mit Hilfe der nachfolgenden Aufnahmen differenzierter betrachtet werden. Der vergrößerte Ausschnitt des Übergangsbereichs zwischen dem Träger gemäß Beispielrezeptur 11 und der Haftklebmasse gemäß Beispielrezeptur 1 zeigt, dass keine Grenze zwischen den Materialien vorhanden ist. Die Trägerschicht besitzt eine Dicke von ca. 2 $\mu$m und ist über eine sichtbare Polymerisationsverankerung von ca. 2 - 3 $\mu$m mit der Klebmasse verbunden.

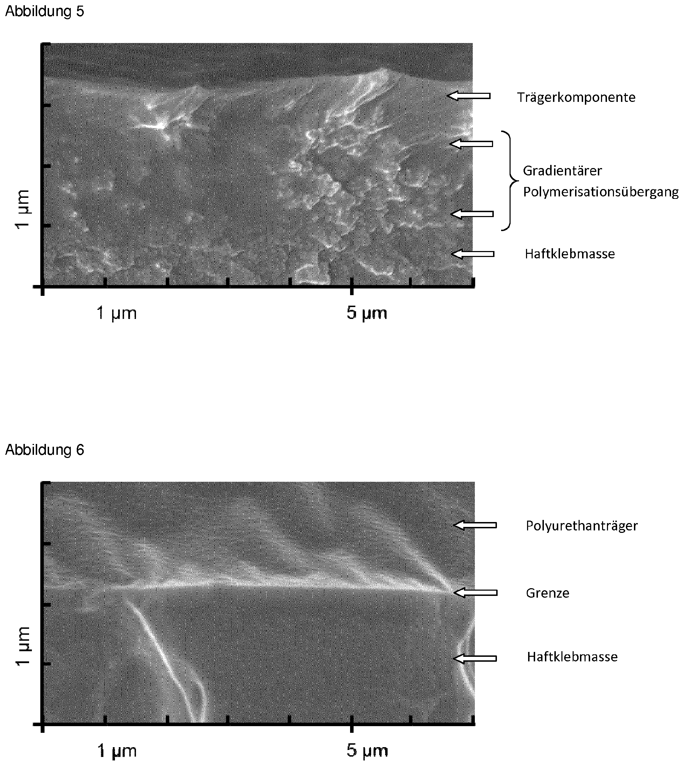

Abbildung 5 zeigt die REM-Vergrößerung des Polymerisationslaminatübergangs im System Beispielrezeptur 1 und 11. Abbildung 5 veranschaulicht die erfindungsgemäße Einpolymerisation der Trägerkomponenten in die Klebmasse. In Abbildung 5 beträgt die Gesamtauflösung der y-Achse nur 5 $\mu$m. Dort kann man dann auch optisch sehr schön darstellen, dass der Übergang zwischen den Materialien keine definierte Grenzschicht aufweist sondern über eine Schichtdicke

von 2 - 3 $\mu$m mäandert, also erfindungsgemäß eine gradientäre Einpolymerisationsschicht ausbildet. Die ist erfindungsgemäß als Einpolymerisation zu verstehen. Zum Unterschied zum Stand der Technik, indem ggf. durch einfaches Aufbringen einer Trägerschicht auf eine Klebmasse es ggf. zu einem molekularen Ineinandergreifen der beiden Schichten kommt, ist es erfindungsgemäß vorteilhaft ein gradientärer Verbund. D.h. die Anzahl oder der Anteil an Trägerkomponenten in Richtung zur Klebmasse nimmt ab und die Anzahl oder der Anteil an Klebmassenkomponenten nimmt in Richtung zum Träger ab. Dies ist erfindungsgemäß als gradientäres einpolymerisieren zu verstehen.

Was wiederum in klarem Kontrast zu Abbildung 6 steht, in der eine herkömmliche Abdeckung einer Haftklebmasse mit einer Polyurethanfolie in der identischen Gesamtauflösung der y-Achse von 5 $\mu$m zu sehen ist.

Die Abbildung 6 zeigt die ganz scharfe Trennung in einem konventionellen Verbundsystem, beispielhaft bestehend aus einer Haftklebmasse acResin 260UV und einem Polyurethan-Träger.

Abbildung 6 zeigt die REM-Vergrößerung des Übergangs zwischen Träger und Klebmasse in einem konventionellen Pflastersystem (acResin 260UV und einem Polyurethan-Träger). Diese Vergleichsbeispiel des Standes der Technik zeigt wie zu erwarten eine lineare Grenzschicht von, optisch abgeschätzt, kleiner 0,1 $\mu$m, also keine gradientäre Einpolymerisationsschicht des Trägers in die Klebmasse.

Zur Anwendung durch den Endverbraucher kann ein erfindungsgemäßes selbstklebendes Polymerisationsprodukt sowohl auf einem Trennpapier als auch auf sich selbst auf Rollen gewickelt, als auch als vorgeformtes flächiges Produkt dargereicht werden. Flächige Produkte können dabei in jedweder denkbaren geometrischen Form als auch Größe ausgeführt sein.

Erfindungsgemäß ist ebenfalls eine Haut- oder Wundauflage gebildet aus einem erfindungsgemäßen Polymerisationsprodukt.

In der besonders bevorzugten Ausführungsform eines erfindungsgemäßen selbstklebenden Polymerisationslaminats als Pflaster, ganz besonders bevorzugt als Wundverband, zur Anwendung auf menschlicher Haut gelten zusätzliche physiko-chemische Anforderungen die von einem erfindungsgemäßen selbstklebenden Polymerisationslaminat ebenfalls hervorragend abgedeckt werden.

Die menschliche Haut gibt stets Wasser in Form von Wasserdampf ab. Bei Verwendung eines Pflasters zur Wundvorbeugung oder -versorgung kann die Hautfeuchtigkeit im Applikationsbereich nicht mehr ungehindert verdunsten.

Unter okklusiven, d.h. wasserdampfundurchlässigen, Pflastern steigt aus diesem Grund die Feuchtigkeit stetig an und bringt die obere Hornschicht der Epidermis zum Quellen. Dies bedingt eine Mazeration, die sich durch die weißliche Verfärbung der Haut zeigt. Zudem kann die Feuchtigkeitsansammlung zum vorzeitigen Ablösen des Pflasters führen. Untersuchungen konnten zeigen, dass der Heilungsprozess einer Wunde im feuchten Wundmilieu um bis zu 50% beschleunigt und zusätzlich die Narbenbildung reduziert werden konnte.

Auch zur Erzeugung und Aufrechterhaltung des feuchten Wundmilieus ist die Wasserdampfdurchlässigkeit eines Pflasters entscheidend. Optimale Wundheilungsbedingungen werden bei einer durchschnittlichen Wasserdampfdurchlässigkeit (water vapour transmission rate, WVTR) von $\leq$ 35 g/m$^2$h erzeugt. (L. Bolton, K. Monte, A. Louis, OstomyWound Management 2000, 46, 51-62; L. Bolton, L. Rijskwijk, J. Dermoatol. Nurs. 1991, 3, 146-161). Zusätzlich kann die WVTR in unterschiedliche Niveaus eingeteilt werden. Als niedrig wird eine WVTR von 11-13 g/m$^2$h, durchschnittlich eine solche von ca. 33 g/m$^2$h und als hoch eine von 67 g/m$^2$h angesehen (L. Bolton, J. Wound Ostomy Continence Nurs. 2007, 34, 23-29).

Vorteilhaft ist es die WVTR eines Pflasters so zu steuern, dass die Haut vor Irritationen geschützt und dennoch die Wunde feucht gehalten werden kann. Deshalb wurde die WVTR der hergestellten erfindungsgemäßen selbstklebenden Polymerisationsprodukte im Vergleich mit einer handelsüblichen semi-okklusiven PU-Trägerfolie untersucht.

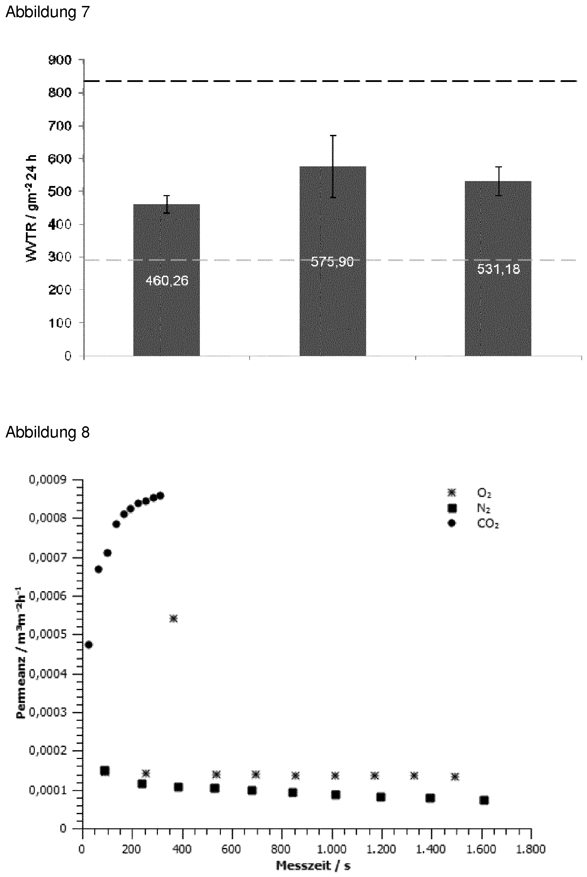

Zur Probenvorbereitung wurde eine Haftklebmatrix gemäß Beispielrezeptur 1 mit einer Dicke von 100 $\mu$m jeweils mit einer Polyurethanfolie kaschiert, bzw. mit einer Trägerkomponente gemäß Beispielrezeptur 66 bzw. einer Trägerkomponente gemäß Beispielrezeptur 67 gradientär einpolymerisiert/laminiert. Die Haftklebmassenseite der Proben wurde zur besseren Testdurchführung jeweils mit einer UV-Dosis von 60 mJ/cm$^2$ nachvernetzt. Die Abbildung 7 zeigt das Ergebnis der WVTR-Messungen der Systeme über 24h.

Die WVTR der beiden erfindungsgemäßen selbstklebenden Polymerisationslaminate sind im Bereich der Standardabweichung vergleichbar und liegen etwas über der WVTR des konventionellen Systems mit PU-Folienträger. Damit befinden sich alle Systeme im optimalen Fenster der WVTR zur Erzeugung und Aufrechterhaltung eines feuchten Wundmilieus.

Bei differenzierter Betrachtung der Systeme zeigt die Formulierung gemäß Beispielrezeptur 1 + 66 eine etwas höhere WVTR als die Formulierung gemäß Beispielrezeptur 1 + 67. Cellulose ist in der Lage eine geringe Menge Wasser zu absorbieren und an die Polymerkette zu binden. Während der Messzeit von 24 h wird somit ein gewisser Teil des Wasserdampfs in der Trägerkomponente des selbstklebenden Polymerisationslaminats zurückgehalten, der erst anschließend an die Umgebung abgegeben werden kann.

Weiterhin nimmt die menschliche Haut durch Diffusion abhängig von der Körperregion zwischen 720 ml/m$^2$ x 24 h und 1872 ml/m$^2$ x 24 h Sauerstoff aus der Atmosphäre auf. (M. Strücker, C. Moll, P. Altmeyer, Der Hautarzt 2004, 3, 273-279,M. Strücker, P. Altmeyer, A. Struk, J. Invest. Dermatol. 2000, 114, 533-540; M. Strücker, A. Struk, P. Altmeyer, J. Physiol. 2002, 538, 985-994). Unterdessen gibt sie zwischen 2160 ml/m$^2$ x 24 h und 2880 ml/m$^2$ x 24 h Kohlenstoffdioxid an die Umgebung ab. Damit unter einem Wundverband kein anaerobes Milieu entsteht, welches die Wundheilung durch Bildung pathogener Keime behindert, müssen die Haut und die Wunde unter dem Pflaster mit Sauerstoff versorgt werden können. Zudem muss das gebildete $CO_2$ abgeführt werden können.

Zur Charakterisierung der erfindungsgemäßen selbstklebenden Polymerisationsprodukte wurden beispielhaft an einem Muster bestehend aus Beispielrezeptur 1 + 66 die Einzelgaspermeanzen für $N_2$, $O_2$ und $CO_2$ per Druckanstiegsmessung ermittelt.

Die Ergebnisse der Permeationsuntersuchungen sind in der Abbildung 8 dargestellt. Abbildung 8 zeigt den Verlauf der Permeanzmessungen der unterschiedlichen Gase mit Hilfe der Druckanstiegsmethode.

Die nachfolgende Tabelle 12 führt die gemessenen Permeanzen und Selektivitäten des Pflastersystems bestehend aus Beispielrezeptur 1 + 66 auf.

|  | Permeanz m$^3$/(m$^2$h bar) | Selektivität |
|---|---|---|
|  |  |  |
| $N_2$ | 7.56 x 10-5 | - |
| $O_2$ | 18.30 x 10-5 | 2.42 |
| $CO_2$ | 83.75 x 10-5 | 11.09 |

Die Ergebnisse zeigen, dass das System bestehend aus Beispielrezeptur 1 + 66 aus einem kompakten, dichten Film besteht. Bei einem porösen System würde die Selektivität entscheidend von der Molekülgröße beeinflusst werden, zudem wären höhere Permeabilitäten erreichbar.

In diesem Fall kann davon ausgegangen werden, dass die Permeation der Gase auf dem Lösungs-Diffusionsprozess in dem Polymersystem beruht. Die deutliche Selektivität des Systems geht somit auf unterschiedliche Sorptions- und Diffusionseigenschaften der Gase im Polymersystem zurück.

Stickstoff ist chemisch inert und zeichnet sich durch seine hohe Stabilität aus. $N_2$ löst sich nur langsam im amorphen Polymersystem und wird nicht über Wechsel-wirkungsphänomene durch das System transportiert. Sauerstoff ist chemisch zugänglicher und weniger stabil als Stickstoff. Das Gas kann sich schneller in der Polymermatrix lösen und demnach schneller durch das System permeieren.

Im Vergleich zur $N_2$-Permeation zeigt ein erfindungsgemäßes selbstklebendes Polymerisationslaminat eine 2.4-mal höhere Selektivität für $O_2$. Die Ergebnisse zeigen zudem eine 11-mal (11,09) höhere Permeationsgeschwindigkeit für $CO_2$ gegenüber $N_2$. Damit ist diese auch 2.5-mal höher als für $O_2$. Die Selektivität erklärt sich durch die reversible Bildung von instabilen Carbamatkomplexen zwischen $CO_2$ und Aminen. In dem System beinhaltend die Trägerkomponente gemäß Beispielrezeptur 66 wird Stickstoff durch die Urethankomponente bereitgestellt. Die Komplexbildung beruht auf der Säure-Base-Wechselwirkung zwischen dem positiv polarisierten Kohlenstoffatom des $CO_2$ und dem freien Elektronenpaar des Stickstoffs. $CO_2$ wird dem Konzentrationsgradienten folgend im Pflastersystem von einer Amidgruppe zur nächsten Amidgruppe geleitet und so durch das System transportiert.

Für die Wundheilung besitzt das Pflastersystem eine gute Sauerstoffdurchlässigkeit (oxygen transmission rate, OTR). Folglich wird die Wundheilung nicht durch anaerobe Bedingungen behindert.

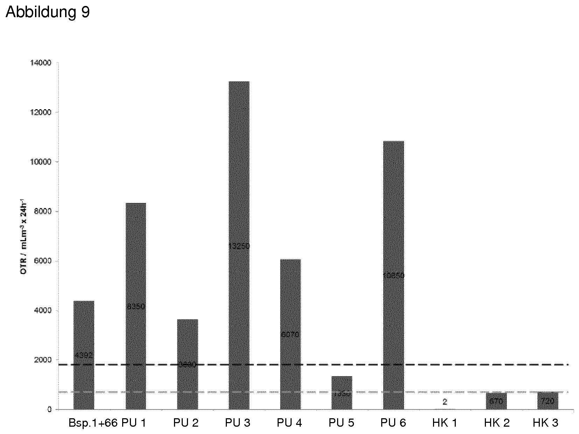

Die Abbildung 9 zeigt einen Vergleich der Permeabilität dieses erfindungsgemäßen Systems bestehend aus Beispielrezeptur 1 + 66 Pflasters zu handelsüblichen Pflastersystemen. Hierbei wurden sechs unterschiedliche Polyurethanpflaster, PU 1 - 6, und drei Hydrokolloidpflaster, HK 1 - 3, untersucht. Die PU-Systeme zeichnen sich durch eine hohe Sauerstoffpermeanz aus. Demnach wird bei allen Systemen die Bildung eines anaeroben Wundmilieus vermieden. Die Hydrokolloidsysteme haben eine deutlich geringere Sauerstoffpermeanz.

Abbildung 9 zeigt den Vergleich der OTR des hergestellten Polymerisationslaminats basierend auf Beispielrezeptur 1 + 66, mit im Handel erhältlichen Produkten. In der Grafik gekennzeichnet ist die Sauerstoffaufnahme der Haut zwischen 720 ml/m$^2$ x 24 h$^{-1}$ und 1872 ml/m$^2$ x 24 h$^{-1}$.

In einfachen Peel-Versuchen zur voll- oder teilfläche Delaminationen der Haftschicht von der Trägerschicht zeigten sich die erfindungsgemäßen Vorteile, indem nämlich eine derartige voll- oder teilfläche Delaminationen nicht möglich ist bzw. nur durch Zerstörung des gesamten Produktes eine Trennung erfolgt. Dabei kann aber nicht mehr von Haft- oder Trägerschichttrennung gesprochen werden.

Auch zeigte sich, dass erfindungsgemäß der kalten Fluss der Haftklebmassen durch den Zusatz von Füllstoffen, wie z. B. Siliziumdioxid oder Cellulose, verbessert wurde ohne das eine Reduktion des Adhäsionsvermögens der Haftklebmasse beobachtet wurde.

Als alternative, jedoch nicht erfindungsgemäße Ausführungsform umfasst die Offenbarung auch ein entsprechendes Laminat, bei dem die Trägerschicht als auch die als "Klebschicht" titulierte Schicht beide nicht klebend formuliert sind. Hier sind, wie zuvor ausgeführt, die als Klebemasse bezeichnete Masse als "nicht-klebend" zu formulieren. Das daraus hergestellte Laminat müsste dann mit zusätzlichen Fixierungen auf einem Substrat befestigt werden.

Weitere Ausführungsformen ergeben sich dann indem mehrere unterschiedliche Laminatschichten, klebend oder nicht klebend, übereinander vernetzen um unterschiedliche Eigenschaften der Schichten miteinander zu kombinieren, oder physiko-chemische Inkompatibilitäten zwischen gewünschten Schichten durch eine gradientär einlaminierte Zwischenschicht zu überbrücken.

D.h. ein erfindungsgemäßen Polymerisationsprodukt besteht aus mehr als zwei Schichten, wobei die Endschicht, die nicht auf der Haut oder einem Substrat appliziert wird, die Trägerschicht darstellt und die Schicht, die auf der Haut oder einem Substrat aufgetragen wird, die Haftklebschicht darstellen. Dazwischen können ein oder mehrere Schichten, umfassend ein oder mehrere polymerisationsfähige Monomere, Oligomere oder Polymere, liegen. Die jeweiligen Schichten werden wie erfindungsgemäß beschrieben aufgetragen und anschließend in die darunter liegende Schicht gradientär einpolymerisiert. Zwischen Träger und Klebmasse sind so ein oder mehrere Schichten polymerisationsfähiger Materialien enthalten, die jeweils auf die darunterliegende Schicht gradientär einpolymerisiert sind. Das erfindungsgemäße Polymerisationsprodukt kann bevorzugt als Hautauflage oder Wundpflaster verwendet werden. Als Hautauflage werden ebenso tapes, patches, pads, dressings oder Bandagen verstanden.

In der besonders bevorzugten Ausprägungsform eines erfindungsgemäßen selbstklebenden Polymerisationslaminats als Wundpflaster bzw. Wundschnellverband wird die Seite des Haftklebers, die zum Substrat (zur Haut) zeigt, zusätzlich mit einer Wundauflage versehen. Diese Wundauflage polstert die Wunde gegen äußerlichen Druck ab und kann exzessives Wundexsudat (z.B. Sekret, Blut) aufnehmen. Die Wundauflage kann dabei aus jedwedem dafür üblichen Material gefertigt sein, wie z.B. Baumwolle, Watte, synthetische oder natürliche Polymerfasern etc. Die Wundauflage kann dabei jedwede Form und Größe annehmen und das Material z.B. gewebt, gesponnen, gepresst, geflockt, als Non-Woven bzw. als Vlies verdichtet vorliegen. Bevorzugt können als Wundauflage auch feuchtigkeitsaufnehmende, selbstklebende oder nicht selbstklebende, hydrokolloidale Polymermatrices verwendet werden.

In einer besonders bevorzugten Ausprägungsform eines Wundpflasters bzw. Wundschnellverbands ist ein erfindungsgemäßes selbstklebendes Polymerisationslaminat mit dünnen, optisch unauffälligen Wundauflagen versehen um insgesamt ein dünnes, unauffälliges und angenehm zu tragendes Endprodukt zu erhalten.

Bei den besonders bevorzugten extrem dünnen Ausprägungsformen eines erfindungsgemäßen selbstklebenden Polymerisationslaminats kann es in Abhängigkeit von den Kohäsivitäten der Haftklebmatrix und der Trägerkomponente bei der Applikation bzw. Entfernung leicht zum Reißen des Produktes kommen. Um ein mögliches Reißen zu verhindern können zur mechanischen Verstärkung des Systems Endlosfasern in die Polymerisationslaminate eingearbeitet werden. Entsprechende Endlosfasern können dabei aus Glas, Kohlenstoff, Natur- und/oder Kunststoffen bestehen. Endlosfasern aus Kunststoffen werden auch als Filamente bezeichnet, diese Bezeichnung findet aber auch immer öfter, wie auch im Nachfolgenden, für die Allgemeinheit von Endlosfasern Anwendung.

Filamente können als Einzelendlosfasern, texturiert oder als Garn, als Kabel, als Multifilgarn oder als Monofilgarn eingesetzt werden.

Bevorzugt werden Filamente in Form von Gitternetzstrukturen eingesetzt. Die Gitterformen können dabei jede beliebige geometrische Form, z.B. quadratisch, rechteckig, oval und/oder rund, sowie jede beliebige Größe und Höhe annehmen

In der besonders bevorzugten Ausführungsform eines erfindungsgemäßen selbstklebendes Polymerisationsproduktes als Haut- oder Wundauflage, ist das Produkt so dünn ausgefertigt, dass eine fast unsichtbare Abdeckung der Wunde oder Haut erfolgt.

Damit scheint jedoch ein Beeinträchtigung verbunden zu sein, da aufgrund der Dünnheit von bevorzugt maximal 300 $\mu$m, bevorzugt kleiner 200 $\mu$m, besonders bevorzugt kleiner 100 $\mu$m, für das Gesamtlaminat eine einwandfreie, entsprechend faltenfreie, Applikation durch den Anwender nicht mehr einfach gewährleistet zu sein.

In diesen Ausführungsformen kann ein erfindungsgemäßes selbstklebendes Polymerisationsprodukt mit einem Stützträger als Applikationshilfe versehen sein. Als Stützträger wird üblicherweise eine durchsichtige Folie, häufig aus Polyethylen, verwendet die auf der bevorzugt nichtklebenden Trägerseite der Auflage anhaftet.

Aber auch Stützträger aus Papier können zum Einsatz kommen. Zur Anwendung wird erst das Trennpapier der Auflage oder des Pflasters entfernt, die Auflage oder das Pflaster dann auf dem Applikationsort verklebt und anschließend der Stützträger von der Auflagenrückseite entfernt. Zur besseren Entfernung kann der Stützträger weiterhin mit einem oder mehreren Hilfselementen versehen sein.

Zu beachten ist, dass der Stützträger besser auf der Trägerseite der Auflage haftet als das Trennpapier bzw. die Trennfolie auf der Haftklebmasse haftet, der Stützträger gleichzeitig schlechter auf der Trägerseite haftet als die Auflage bzw. die Haftklebmasse der Auflage auf dem vorgesehenen Substrat (dem Applikationsort).

Diese Haftungsvorgaben können durch Folien erreicht werden, die über elektrostatische Anziehungskräfte an den Träger des Laminats gebunden sind. In einer anderen Ausführungsform kann die Stützfolie selbst mit einer sehr schwach adhäsiven Klebmasse beschichtet sein und auf diese Weise einen entsprechenden Verbund erzeugen.

In einer bevorzugten Ausführungsform werden die Auflagen, Pflaster bzw. Wundschnellverbände, hergestellt aus einem erfindungsgemäßen selbstklebenden Polymerisationslaminat, als Endprodukt einzeln zwischen zwei Folien eingesiegelt dargeboten. In einer besonders bevorzugten Ausführungsform ist zumindest eine der beiden Siegelfolien durchsichtig und fungiert gleichzeitig als Stützträger zur Applikation.

Wie bereits dargestellt, stellt die Unauffälligkeit eines erfindungsgemäßen beschriebenen Polymerisationslaminats einen besonderen Vorteil der erfindungsgemäßen Produkte dar, insbesondere bei einer Pflasteranwendung am Menschen. Die Unauffälligkeit begründet sich sowohl in der geringen Schichtdicke wie auch in der möglichen Transparenz der Schichten.

Bei manchen Indikationen zur Pflasteranwendung am Menschen kann allerdings eine farbliche Kaschierung oder Abdeckung des Anwendungsareals erwünscht bzw. vorteilhaft sein. Dies gilt z.B. insbesondere zur Abdeckung von Hautirritationen oder Pigmentstörungen wie Altersflecken, Narbengewebe etc. Für solcherart Anwendung eines finalen selbstklebenden Polymerisationslaminats können die Produkte in der Herstellung eingefärbt werden. Dies kann durch Zugabe von Farbstoffen oder Pigmenten erfolgen und jedwede Färbung beinhalten. Die Zugabe der Färbemittel kann dabei sowohl in das Haftklebemittel, als auch als Bestandteil der Trägerkomponente erfolgen.

Bei Bedarf können auch beide Komponenten des Polymerisationslaminats eingefärbt werden. Dies sowohl mit gleichartigen und -farbigen bzw. identischen Farbstoffen oder Pigmenten, als auch zur Erzielung besonderer farblicher Effekte mit jeweils unterschiedlichen Substanzen.

## Patentansprüche

1. Selbstklebendes Polymerisationsprodukt umfassend eine Klebmasse und einen darauf aufgebrachten nicht-klebenden Träger, der vor der Polymerisation ein oder mehrere polymerisationsfähige Monomere, Oligomere und/oder Polymere umfasst, die teilweise und/oder vollständig in die Klebmasse einpolymerisiert sind, **dadurch gekennzeichnet, dass** das Polymerisationsprodukt eine maximale Dicke von bis zu 800 $\mu$m aufweist und die Schicht der Klebmasse, die gradientäre Übergangsschicht in der die Monomere, Oligomere und/oder Polymere des Trägers teilweise und/oder vollständig einpolymerisiert sind, eine Tiefe von bis zu 100 $\mu$m (rasterelektronenmikroskopisch REM) aufweist.

2. Polymerisationsprodukt nach Anspruch 1 **dadurch gekennzeichnet, dass** die Schichttiefe 1 $\mu$m bis maximal 50 $\mu$m beträgt.

3. Polymerisationsprodukt nach einem der vorstehenden Ansprüche **dadurch gekennzeichnet, dass** die Polymerisation durch UV-Lichtbestrahlung mit 10 bis 200 mJ/cm$^2$, bevorzugt 120 bis 150 mJ/cm$^2$ erfolgt.

4. Polymerisationsprodukt nach einem der vorstehenden Ansprüche **dadurch gekennzeichnet, dass** die Klebmasse UV-vernetzbare Acrylathaftklebstoffe umfasst oder vollständig daraus besteht.

5. Polymerisationsprodukt nach einem der vorstehenden Ansprüche **dadurch gekennzeichnet, dass** die Klebmasse ein oder mehrere Stoffe ausgewählt aus der Gruppe der Füllstoffe, insbesondere Siliziumdioxid und Cellulose, *cis*-1,4-Polyisopren, Polyisobutylen sowie ein oder mehrere kosmetische, dermatologische oder pharmazeutische Wirkstoffe enthält.

6. Polymerisationsprodukt nach einem der vorstehenden Ansprüche **dadurch gekennzeichnet, dass** als Träger Pentaerythritoltetraacrylate, Butylacrylate, Polyethylenglycoldiacrylate, Pentaerytritholtriacrylate, Polyurethanacrylate sowie Polyesteracrylate oder Mischungen daraus verwendet werden.

7. Polymerisationsprodukt nach einem der vorstehenden Ansprüche **dadurch gekennzeichnet, dass** der Träger Polyethylenglycoldiacrylat, Pentaerytritholtriacrylat, Polyurethanacrylate und/oder Polyesteracrylate umfasst.

8. Polymerisationsprodukt nach einem der vorstehenden Ansprüche **dadurch gekennzeichnet, dass** der Träger Thiole umfasst.

9. Polymerisationsprodukt nach einem der vorstehenden Ansprüche **dadurch gekennzeichnet, dass** die Polymerisation radikalisch erfolgt.

10. Polymerisationsprodukt nach einem der vorstehenden Ansprüche mit einer Dicke von maximal 350 $\mu$m, insbesondere mit einer Dicke im Bereich von 20 bis 250 $\mu$m.

11. Hautauflage umfassend oder bestehend aus einem Polymerisationsprodukt nach einem der vorstehenden Ansprüche.

12. Verwendung des Polymerisationsproduktes nach einem der vorstehenden Ansprüchen 1 bis 10 als Hautauflage oder Wundpflaster.

13. Verfahren zur Herstellung eines selbstklebenden Polymerisationsproduktes nach einem der vorstehenden Ansprüche 1 bis 10 **gekennzeichnet durch** die Schritte der Applikation eines Trägermaterials, umfassend ein oder mehrerer polymerisationsfähige Monomere, Oligomere und/oder Polymere, sowie ggf. weitere Zusatzstoffe, bevorzugt in Lösung, auf eine Schicht eines ggf. teilweise vorvernetzten Haftklebstoffes, umfassend bevorzugt UV-vernetzbare Acrylathaftklebstoffe, Photoinitiatoren sowie ggf. weitere Zusatzstoffe, und Polymerisation der Haftkleb-Trägerschichten mittels UV-Lichtbestrahlung, bevorzugt mit 10 bis 200 mJ/cm$^2$, bevorzugt 120 bis 150 mJ/cm$^2$.

14. Verfahren nach Anspruch 13 **dadurch gekennzeichnet, dass** die Polymerisation durch freie radikalische Polymerisation erfolgt.

**Claims**

1. Self-adhesive polymerization product comprising an adhesive composition and a non-adhesive carrier applied thereto and comprising before polymerization one or more polymerizable monomers, oligomers and/or polymers which are incorporated partially and/or entirely into the adhesive composition by polymerization, **characterized in that** the polymerization product has a maximum thickness of up to 800 $\mu$m and the layer of the adhesive composition, the gradientary transition layer with a gradient in which the monomers, oligomers and/or polymers of the carrier are partially and/or completely incorporated by polymerization, has a depth of up to 100 $\mu$m by scanning electron microscopy (SEM).

2. Polymerization product according to Claim 1, **characterized in that** the layer depth is 1 $\mu$m to not more than 50 $\mu$m.

3. Polymerization product according to either of the preceding claims, **characterized in that** the polymerization takes place by UV light irradiation with 10 to 200 mJ/cm$^2$, preferably 120 to 150 mJ/cm$^2$.

4. Polymerization product according to any of the preceding claims, **characterized in that** the adhesive composition comprises UV-crosslinkable pressure-sensitive acrylate adhesives or consists completely thereof.

5. Polymerization product according to any of the preceding claims, **characterized in that** the adhesive composition comprises one or more substances selected from the group of fillers, especially silicon dioxide and cellulose, *cis*-1,4-polyisoprene, polyisobutylene and also one or more active cosmetic, dermatological or pharmaceutical ingredients.

6. Polymerization product according to any of the preceding claims, **characterized in that** carriers used comprise pentaerythritol tetraacrylates, butyl acrylates, polyethylene glycol diacrylates, pentaerythritol triacrylates, polyurethane acrylates and also polyester acrylates or mixtures thereof.

7. Polymerization product according to any of the preceding claims, **characterized in that** the carrier comprises polyethylene glycol diacrylate, pentaerythritol triacylate, polyurethane acrylates and/or polyester acrylates.

8. Polymerization product according to any of the preceding claims, **characterized in that** the carrier comprises thiols.

9. Polymerization product according to any of the preceding claims, **characterized in that** the polymerization takes place radically.

10. Polymerization product according to any of the preceding claims, having a thickness of not more than 350 $\mu$m, more particularly having a thickness in the range from 20 to 250 $\mu$m.

11. Skin dressing comprising or consisting of a polymerization product according to any of the preceding claims.

12. Use of the polymerization product according to any of preceding Claims 1 to 10 as a skin dressing or wound plaster.

**13.** Method for producing a self-adhesive polymerization product according to any of preceding Claims 1 to 10, **characterized by** the steps of applying a carrier material comprising one or more polymerizable monomers, oligomers and/or polymers, and also, optionally, further adjuvants, preferably in solution, to a layer of an optionally partially precrosslinked pressure-sensitive adhesive, comprising preferably UV-crosslinkable pressure-sensitive acrylate adhesives, photoinitiators and also, optionally, further adjuvants, and polymerizing the pressure-sensitive adhesive carrier layers by means of UV light irradiation, preferably with 10 to 200 mJ/cm$^2$, preferably 120 to 150 mJ/cm$^2$.

**14.** Method according to Claim 13, **characterized in that** the polymerization takes place by free radical polymerization.

**Revendications**

**1.** Produit de polymérisation auto-adhésif comprenant une masse adhésive et un support non adhésif appliqué sur celle-ci, qui comprend avant la polymérisation un ou plusieurs monomères, oligomères et/ou polymères polymérisables, qui sont polymérisés en partie et/ou en totalité dans la masse adhésive, **caractérisé en ce que** le produit de polymérisation présente une épaisseur maximale de jusqu'à 800 um et la couche de masse adhésive, la couche de transition en gradient, dans laquelle les monomères, oligomères et/ou polymères du support sont polymérisés en partie et/ou en totalité présente une profondeur de jusqu'à 100 um (microscopie électronique à balayage, MEB).

**2.** Produit de polymérisation selon la revendication 1, **caractérisé en ce que** la profondeur de couche est de 1 um à au plus 50 um.

**3.** Produit de polymérisation selon l'une quelconque des revendications précédentes, **caractérisé en ce que** la polymérisation a lieu par exposition à de la lumière UV avec 10 à 200 mJ/cm$^2$, de préférence 120 à 150 mJ/cm$^2$.

**4.** Produit de polymérisation selon l'une quelconque des revendications précédentes, **caractérisé en ce que** la masse adhésive comprend des adhésifs sensibles à la pression à base d'acrylates réticulables par UV ou en est entièrement constituée.

**5.** Produit de polymérisation selon l'une quelconque des revendications précédentes, **caractérisé en ce que** la masse adhésive contient une ou plusieurs substances choisies dans le groupe constitué par les charges, notamment le dioxyde de silicium et la cellulose, le *cis*-1,4-polyisoprène, le polyisobutylène, ainsi qu'un ou plusieurs agents actifs cosmétiques, dermatologiques ou pharmaceutiques.

**6.** Produit de polymérisation selon l'une quelconque des revendications précédentes, **caractérisé en ce que** des tétraacrylates de pentaérythritol, des acrylates de butyle, des diacrylates de polyéthylène glycol, des triacrylates de pentaérythritol, des acrylates de polyuréthane et des acrylates de polyester ou leurs mélanges sont utilisés en tant que support.

**7.** Produit de polymérisation selon l'une quelconque des revendications précédentes, **caractérisé en ce que** le support comprend du diacrylate de polyéthylène glycol, du triacrylate de pentaérythritol, des acrylates de polyuréthane et/ou des acrylates de polyester.

**8.** Produit de polymérisation selon l'une quelconque des revendications précédentes, **caractérisé en ce que** le support comprend des thiols.

**9.** Produit de polymérisation selon l'une quelconque des revendications précédentes, **caractérisé en ce que** la polymérisation a lieu par voie radicalaire.

**10.** Produit de polymérisation selon l'une quelconque des revendications précédentes, ayant une épaisseur d'au plus 350 $\mu$m, notamment ayant une épaisseur dans la plage allant de 20 à 250 $\mu$m.

**11.** Compresse pour la peau, comprenant ou constituée par un produit de polymérisation selon l'une quelconque des revendications précédentes.

**12.** Utilisation du produit de polymérisation selon l'une quelconque des revendications 1 à 10 en tant que compresse pour la peau ou pansement pour plaies.

**13.** Procédé de fabrication d'un produit de polymérisation auto-adhésif selon l'une quelconque des revendications 1 à 10 précédentes, **caractérisé par** les étapes d'application d'un matériau support, comprenant un ou plusieurs monomères, oligomères et/ou polymères polymérisables, ainsi qu'éventuellement d'autres additifs, de préférence en solution, sur une couche d'un adhésif sensible à la pression éventuellement partiellement pré-réticulé, comprenant de préférence des adhésifs sensibles à la pression à base d'acrylates réticulables par UV, des photoinitiateurs, ainsi qu'éventuellement d'autres additifs, et de polymérisation des couches adhésif sensible à la pression-support par exposition à de la lumière UV, de préférence avec 10 à 200 mJ/cm$^2$, de préférence 120 à 150 mJ/cm$^2$.

**14.** Procédé selon la revendication 13, **caractérisé en ce que** la polymérisation a lieu par polymérisation radicalaire libre.

Abbildung 1

Abbildung 2

Abbildung 3

Nicht-adhäsive Oberfläche
Trägerkomponente
Haftklebmasse

Abbildung 4

Polyurethanträger

Haftklebmasse

Abbildung 5

Trägerkomponente

Gradientärer
Polymerisationsübergang

Haftklebmasse

1 µm

1 µm      5 µm

Abbildung 6

Polyurethanträger

Grenze

Haftklebmasse

1 µm

1 µm      5 µm

Abbildung 7

Abbildung 8

Abbildung 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0446431 A2
- WO 9742922 A1
- EP 1064149 B1
- DE 3779707 T2
- EP 259094 A1
- DE 10134261 A1, R. Fink, K.-H. Schumacher, U. Düsterwald, U. Erhardt, H. Meyer

**In der Beschreibung aufgeführte Nicht-Patentliteratur**

- **G. D. WINTER.** *Nature,* 1962, vol. 193, 293-294
- **C.E. HOYLE.** Radiation curing of polymeric materials. American Chemical Society, 1990, 1-16
- **C. NASON ; T. ROPER ; C. HOYLE ; J.A. POJMAN.** *Macromolecules,* 2005, vol. 38, 5506-5512
- **L. BOLTON ; K. MONTE ; A. LOUIS.** *OstomyWound Management,* 2000, vol. 46, 51-62
- **L. BOLTON ; L. RIJSKWIJK.** *J. Dermoatol. Nurs.,* 1991, vol. 3, 146-161
- **L. BOLTON.** *J. Wound Ostomy Continence Nurs.,* 2007, vol. 34, 23-29
- **M. STRÜCKER ; C. MOLL ; P. ALTMEYER.** *Der Hautarzt,* 2004, vol. 3, 273-279
- **M. STRÜCKER ; P. ALTMEYER ; A. STRUK.** *J. Invest. Dermatol.,* 2000, vol. 114, 533-540
- **M. STRÜCKER ; A. STRUK ; P. ALTMEYER.** *J. Physiol.,* 2002, vol. 538, 985-994